(19) Europäisches Patentamt
European Patent Office
Office européen des brevets

(11) Publication number: **0 290 922 B1**

(12) **EUROPEAN PATENT SPECIFICATION**

(45) Date of publication of patent specification: **10.11.93**

(51) Int. Cl.5: **C07K 9/00**, A61K 37/02

(21) Application number: **88107069.2**

(22) Date of filing: **03.05.88**

(54) **Teicoplanin derivatives.**

(30) Priority: **11.05.87 GB 8711066**

(43) Date of publication of application:
**17.11.88 Bulletin 88/46**

(45) Publication of the grant of the patent:
**10.11.93 Bulletin 93/45**

(84) Designated Contracting States:
**AT BE CH DE ES FR GB GR IT LI LU NL SE**

(56) References cited:
**EP-A- 0 146 053**
**EP-A- 0 152 902**
**EP-A- 0 195 359**
**EP-A- 0 201 251**
**WO-A-86/00076**
**WO-A-87/03285**

(73) Proprietor: **GRUPPO LEPETIT S.p.A.**
**Via Roberto Lepetit, 8**
**I-20020 Lainate (MI)(IT)**

(72) Inventor: **Malabarba, Adriano**
**5/A Via Roma**
**I-20082 Binasco (MI)(IT)**
Inventor: **Trani, Aldo**
**11, Via Longhi**
**I-20137 Milano(IT)**
Inventor: **Tarzia, Giorgio**
**3, Vicolo San Clemente**
**I-37100 Verona(IT)**

(74) Representative: **Sgarbi, Renato**
**GRUPPO LEPETIT S.p.A.**
**Patent and Trademark Department**
**Via Roberto Lepetit, 34**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention is directed to new antibiotic substances which are 34-de-(acetylaminoglucopyranosyl)-34-deoxy teicoplanin derivatives of the following formula I

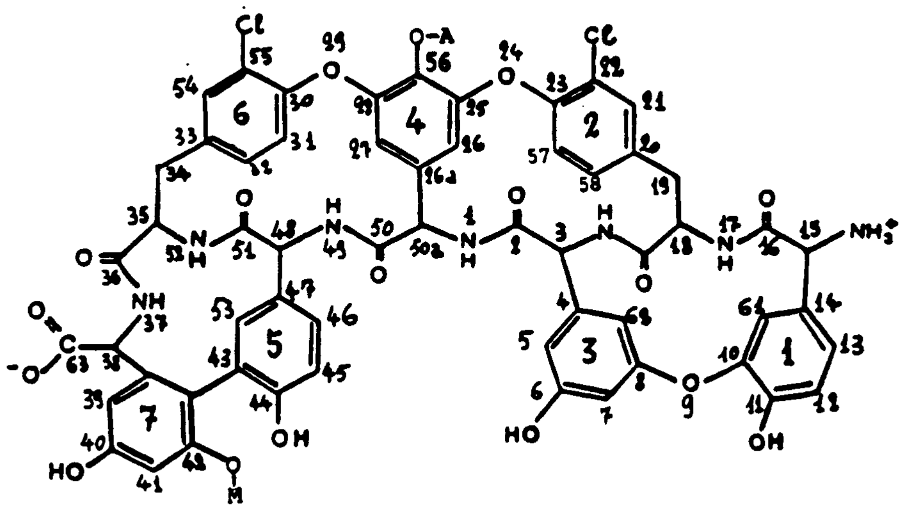

I

wherein

A represents hydrogen or OA represents N[($C_{10}$-$C_{11}$)aliphatic acyl]-beta-D-2-deoxy-2-aminoglucopyranosyl,

M represents hydrogen or OM represents alpha-D-mannopyranosyl, with the proviso that when M represents hydrogen also A must represent hydrogen,

and the addition salts thereof.

In the present disclosure and claims "($C_{10}$-$C_{11}$)aliphatic acyl" represents a saturated or unsaturated aliphatic acyl group of 10 or 11 carbon atoms. Preferred examples of ($C_{10}$-$C_{11}$)aliphatic acyl groups are: (Z)-4-decenoyl, 8-methylnonanoyl, decanoyl, 8-methyldecanoyl, and 9-methyldecanoyl.

When A and M represent sugar moieties as above defined, they are linked to the core moiety through O-glycosidic bonds and all the amide bonds are in the transoid orientation.

The compounds of the invention possess antimicrobial activity, mainly against gram positive bacteria, some coagulase-negative staphylococci included.

They are prepared either by reductive displacement under selective conditions of the sugar unit at position 34 of teicoplanin, a teicoplanin factor, component, pseudoaglycon or derivative thereof or by catalytic hydrogenation of a suitable 34-de-(acetylaminoglucopyranosyl)-34-deoxy-35,52-didehydro-teicoplanin derivative.

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751). According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column chromatography on Sephadex®.

British Patent Application Publication No. 2121401 discloses that antibiotic Teichomycin $A_2$ actually is a mixture of five closely related co-produced main components.

According to recent structural studies it is possible to represent teicoplanin $A_2$ (formerly Teichomycin $A_2$) main components 1, 2, 3, 4 and 5 by the following formula II

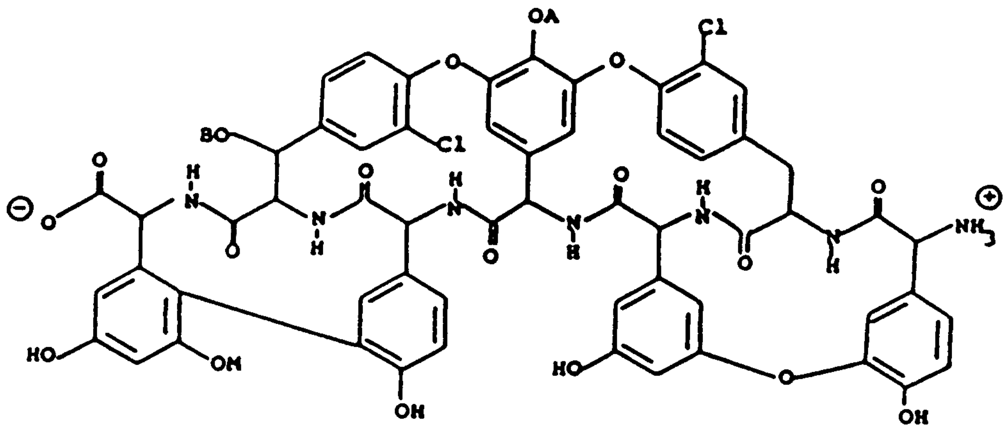

II

wherein OA represents -N[($C_{10}$-$C_{11}$)aliphatic acyl]-beta-D-2-deoxy-2-aminoglucopyranosyl, OB represents N-acetyl-beta-D-2-deoxy-2-amino-glucopyranosyl, OM represents alpha-D-mannopyranosyl. More particularly in teicoplanin $A_2$ component 1, the [($C_{10}$-$C_{11}$)-aliphatic acyl] substituent represents (Z)-4-decenoyl, in teicoplanin $A_2$ component 2 represents 8-methylnonanoyl, in teicoplanin $A_2$ component 3 represents decanoyl, in teicoplanin $A_2$ component 4 represents 8-methyldecanoyl, and in teicoplanin $A_2$ component 5 represents 9-methyldecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties. They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70 ° C and 90 ° C and for a time which is generally between 15 and 90 min.

Antibiotic L 17054 is represented by the above formula II wherein A represents hydrogen, OB represents N-acetyl-beta-D-2-deoxy-2-amino-glucopyranosyl, OM represents alpha-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50 ° and 90 ° C and for a time which is generally between 30 and 60 min.

Antibiotic L 17046 is represented by the above formula II wherein A and M represent hydrogen atoms, and OB is N-acetyl-beta-D-2-deoxy-2-amino-glucopyranosyl wherein the sugar moiety is linked to the peptidic nucleus through an O-glycosidic bond.

The complete selective cleavage of all the sugar moieties of the teicoplanin compounds gives an aglycone molecule which is called antibiotic L 17392, or deglucoteicoplanin, and is represented by the above formula II wherein A, B, and M each individually represents a hydrogen group. This selective hydrolysis process is described in European patent application Publication No. 146053. Deglucoteicoplanin is also prepared through a process that involves treating a corresponding ester derivative under catalytic hydrogenation conditions, as described in W086/00076.

A substance having the same structural formula as deglucoteicoplanin is disclosed in European Patent Application Publication No. 0090578 and is named antibiotic A 41030 factor B.

This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

Some of the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, and any mixture thereor in any proportion, are suitable starting materials for the preparation of the derivatives of the invention.

34-De-(acetylaminoglucopyranosyl)-34-deoxy-35,52-didehydro teicoplanin derivatives which can be used as starting materials in the process of the invention are represented by the following formula III

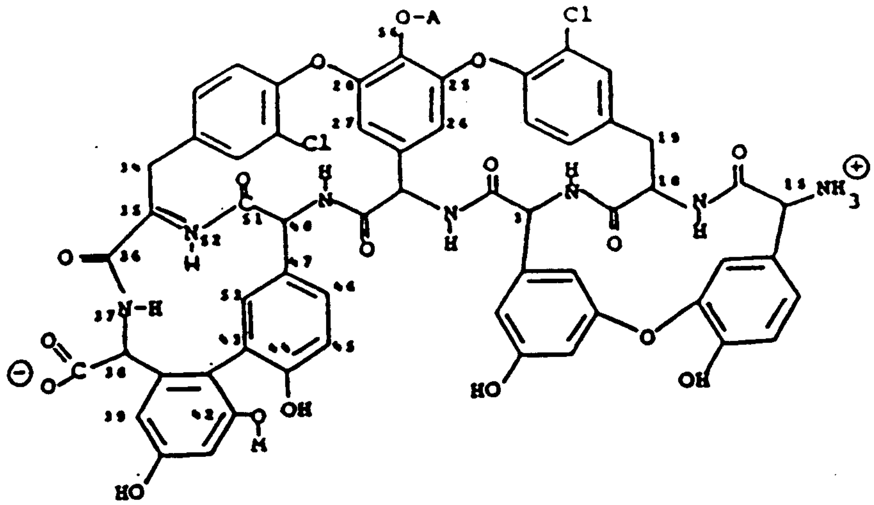

III

wherein

A represents hydrogen or OA represents N[($C_{10}$-$C_{11}$)aliphatic acyl]-beta-D-2-deoxy-2-amino-glucopyranosyl,

M represents hydrogen or OM represents alpha-D-mannopyranosyl, with the proviso that when M represents hydrogen also A must represent hydrogen, and the addition salts thereof.

These compounds and their preparation have been described in WO87/703285 that was published after the filing date of this application.

As stated above, the compounds of the invention may be prepared either by reductive displacement of the sugar unit at position 34 of teicoplanin, a teicoplanin factor, component, pseudoaglycon or derivative thereof or by catalytic hydrogenation of a corresponding 34-de-(acetylaminoglucopyranosyl)-34-deoxy-35,52-didehydro-teicoplanin derivative of formula III.

More particularly, the catalytic hydrogenation of a corresponding 34-de-(acetylaminoglucopyranosyl)-34--deoxy-35,52-didehydro teicoplanin derivative of formula III is conducted in an aqueous alcoholic medium preferably at room temperature and pressure.

Representative examples of catalysts that may be employed in this hydrogenation step are the usual hydrogenation catalysts such as transition metals or transition metal derivatives, e.g. oxides, generally on suitable supports such as palladium on barium sulphate palladium on charcoal, palladium on alumina, palladium on calcium carbonate, platinum, platinum oxide, rhodium on charcoal and the like. A particularly preferred hydrogenation catalyst is palladium on charcoal and most preferably 5% palladium on charcoal. The reaction medium must be compatible with the catalyst as well as the starting material and the final product and is preferably an acidified aqueous alcoholic medium. In some instances however, a slightly basic medium is required by the catalyst, as in the case of a basic support such as alumina or $CaCO_3$. In this case, the reaction medium may conveniently include an alkyl amine like triethylamine, or other non-reactive amines. In addition to 5% palladium on charcoal, 10% palladium on charcoal, 10% palladium on barium sulfate, 5% palladium on calcium carbonate, 5% rhodium on charcoal can also be cited as suitable catalysts.

A preferred acidic aqueous alcoholic medium is represented by a mixture of water and a water soluble lower alkanol, preferably of one to four carbon atoms, in the presence of a mineral acid such as a hydrohalidic acid.

Preferably hydrochloric acid is employed as the hydrohalidic acid while particularly preferred lower alkanols are methanol and ethanol and most preferably methanol. In general, the aqueous alcoholic mixture is a homogeneous mixture wherein the alcohol represents the major component. Preferred mixtures are from 1:1 to 3:1. A preferred mixture is a 70:30, alkanol:acidic water mixture. A further preferred mixture is a 70:30 mixture of methanol (or ethanol) and 0.04 N hydrochloric acid. The reaction temperature is generally between 20 °C and 60 °C, more conveniently room temperature.

The pressure may be varied depending on the other reaction parameters such as type and concentration of the catalyst and temperature, and may vary from ambient pressure to 5 atm (0.5 MPa). Usually an excess of hydrogen is necessary to complete the reaction which may be followed by monitoring the decrease of the starting material by NMR (disappearance of the peaks related to the 35,52 double bond) or by observing by HPLC that no relevant transformation in a more lipophilic product is obtained after treating

a sample with a mild reducing agent.

Examples of this reducing agent are metals capable of developing hydrogen from an aqueous acid solution such as zinc or tin in hydrochloric acid or other substances having a similar standard reduction potential in the electrochemical series.

This pre-treatment is necessary because a compound of the invention and the corresponding 35,52-unsaturated intermediate have a very similar mobility in chromatographic systems and a mixture of them can be analyzed or separated effectively only by the above procedure.

After removing the insolubles, by filtration or by other convenient means, the desired product is isolated and purified according to known per se techniques. For example the filtrate may be concentrated to a small volume and the product may be precipitated by adding a non-solvent, and further purified, if necessary, by crystallization or chromatography.

Probably, the only drawback of the above catalytic hydrogenation procedure is that when teicoplanin $A_2$ component 1 is a starting material it is not transformed into the corresponding compound of formula I wherein $(C_{10}$-$C_{11})$aliphatic acyl represents (Z)-4-decenoyl but into the corresponding compound of formula I wherein $(C_{10}$-$C_{11})$aliphatic acyl represents decanoyl.

In other words, under these conditions, also the double bond on the N-acyl substituent of the glucosamine moiety at position 56 is reduced. Therefore, to prepare a compound of formula I wherein the $(C_{10}$-$C_{11})$aliphatic acyl is an unsaturated group another reaction pathway is to be followed.

This includes selectively removing the N-acetyl-beta-D-2-deoxy-2-aminoglucopyranosyl group from teicoplanin $A_2$ component 1 or from a mixture containing it, like teicoplanin $A_2$, under conditions which do not affect the unsaturation on the acyl chain, such as in the presence of a molar excess of an alkali metal or alkali-earth metal borohydride, in a suitable reaction medium. Representative examples of alkali metal or alkali-earth metal borohydrides are lithium, sodium, potassium or calcium borohydride and sodium cyanoborohydride. The temperature may be between 15 °C and 60 °C but preferably is room temperature. The solvent is a mixture of a polar aprotic solvent and a polar protic solvent. This mixture is preferably a 1:1 mixture but also mixtures from 0.5:1.5 to 1.5:0.5 may be used.

Representative examples of polar aprotic solvents are organic amides such as dimethylformamide and diethylformamide, phosphoramides such as hexamethylphosphoramide, sulfoxides such as dimethylsulfoxide and diethylsulfoxide and the like.

Representative examples of polar protic solvents are lower alkanols, glycols and their ethers and esters, such as methanol, ethanol, propanol, ethyleneglycol, propyleneglycol, methoxyethanol and the like.

A preferred solvent mixture is a mixture dimethylformamide:methanol, 1:1 (v/v). The reaction time depends obviously on the other reaction parameters and is, in general, between 24 and 96 h at room temperature. The reaction course may be followed by NMR or HPLC after treatment with a mild reducing agent as indicated above.

The obtained reaction mixture contains the 35,52-unsaturated compounds of formula III corresponding to the starting material as well as the corresponding saturated compound of formula I (wherein the unsaturations of the $(C_{10}$-$C_{11})$aliphatic acyl chain, if present, are unaffected) which, upon isolation of the solid which forms by working it up according to known per se isolation techniques, is treated with a mild reducing agent, as indicated above to give the desired compound of formula I. As already said, this mild reducing agent, which selectively transforms 35,52-unsaturated compounds of formula III into more lipophilic substances but does not affect compounds of formula I, is preferably a metal capable of developing hydrogen from an aqueous acid solution such as Zn or Sn or other substances having similar standard reduction potential in the electrochemical series. The reducing agent is used in a molar excess and the temperature is generally between 10 °C and 60 °C, preferably between 15 °C and 30 °C and conveniently is room temperature. The desired compound of formula I is then obtained by known per se isolation techniques. For example, after having filtered the insolubles, the aqueous mixture is extracted with a solvent scarcely miscible with water such as an alcohol of four or more carbon atoms, e.g. n-butanol. The organic layer is adjusted to neutral pH and concentrated to a small volume. Precipitation with a non-solvent, e.g. ethyl ether, gives a solid which is dissolved in a suitable solvent mixture such as acetonitrile:water 2:8 (v/v).

The pH of this solution is then brought to an acidic value, preferably between 2 and 4.5 and most preferably about 3 and then it is passed through a reverse-phase silica-gel column to separate the desired product of formula I from the more lipophilic by-product.

An example of a preferred eluting mixture is a mixture of water and a polar aprotic solvent such as acetonitrile.

The above described displacement procedure employing a metal borohydride is also convenient for preparing an intermediate compound of formula III, in admixture with the corresponding compound of formula I, that can be conveniently reduced to the corresponding compound of formula I, with fairly good

yields without previous isolation or purification, by catalytic hydrogenation.

In particular, if either the substrate is different from teicoplanin $A_2$ component 1 or if teicoplanin $A_2$ component 1 is present but a compound of formula I wherein ($C_{10}$-$C_{11}$)aliphatic acyl represents decanoyl instead of (Z)-4-decenoyl is desired, it can be obtained by the catalytic hydrogenation described above when dealing with the reduction of an intermediate compound of formula III. The above obtained mixture can thus be catalytically hydrogenated as such without a previous purification step. The desired compound of formula I can be then isolated according to known per se techniques which include extraction with solvents, precipitation with non-solvents and purification by column chromatography and reverse-phase column chromatography in particular, as described above.

Alternatively, the compounds of formula III can be prepared under selective basic conditions such that the removal of the N-acetyl-beta-D-2-deoxy-2-amino-glucopyranosyl group from the teicoplanin starting material takes place without simultaneously affecting the other sugar moieties, when present, and without causing any substantial epimerization of the core peptide. The term "teicoplanin starting material" is used to indicate any one of the above mentioned starting materials, i.e. teicoplanin as obtained according to U.S. patent 4,239,751, any further purification thereof, teicoplanin $A_2$ complex, a compound of formula II wherein A represents hydrogen or OA represents -N[($C_{10}$-$C_{11}$)aliphatic acyl]-beta-D-2-deoxy-2-amino-glucopyranosyl wherein ($C_{10}$-$C_{11}$)aliphatic acyl has the above defined meanings, B represents hydrogen or OB represents N-acetyl-beta-D-2-deoxy-2-amino-glucopyranosyl, M represents hydrogen or OM represents alpha-D-mannopyranosyl, with the proviso that A, B and M may not simultaneously represent hydrogen and M represents hydrogen only when A represents hydrogen, and a salt thereof, or a mixture thereof in any proportion.

Said selective basic conditions are represented by a strong alkali in a polar organic solvent at a temperature lower than about 60 °C.

Representative examples of polar organic solvents are lower alkanols, lower alkyl carboxamides, lower alkyl sulfoxamides, lower alkyl phosphoramides, lower alkyl sulfoxides and lower alkyl sulfones and the like and mixtures thereof.

Lower alkanols as above described are alkanols of 1 to 4 carbon atoms including methanol, ethanol, propanol, 1-methylethanol, butanol and 2-methyl-propanol.

The term "lower alkyl" as used above represents alkyl groups of 1, 2, 3 or 4 carbon atoms.

Examples of lower alkyl carboxamides are dimethylformamide, diethylformamide and the like. A preferred lower alkyl sulfoxide is dimethylsulfoxide, a preferred lower alkyl sulfone is dimethylsulfone and a preferred lower alkyl phosphoramide is hexamethylphosphoramide.

According to a preferred embodiment of this process, the polar organic solvent as above defined is a mixture of a polar aprotic organic solvent and a polar protic organic solvent. Among those solvents defined above, the preferred polar aprotic solvents are tertiary alkyl amides and dialkyl sulfoxides and sulfones, while the preferred polar protic organic solvents are lower alkanols.

As stated above, the basic conditions required for the displacement of the N-acetyl-D-2-deoxy-2-amino-glucopyranosyl group from the starting material are obtained by means of strong alkali. Preferred examples of said strong alkali are concentrated aqueous alkali metal hydroxides, alkali metal oxides and alkali metal alkoxides of 1, 2, 3 or 4 carbon atoms. The alkali metals are preferably sodium or potassium, and the preferred alkoxy groups are methoxy, ethoxy, propoxy, and butoxy.

When the base is represented by an alkali metal alkoxide, the polar organic solvent is preferably the corresponding alkanol, possibly in a mixture with a polar aprotic solvent as defined above. For efficiently performing this process, the reaction environment must contain a limited amount of water. In general, this is already present in the starting materials which in many instances are hydrates.

When the strong alkali is an alkali hydroxide or oxide, an amount of water of about from 0.5 to 2% (w/w), or a 15-20 molar excess, is highly preferred. Higher amounts of water negatively interfere with the reaction course by favouring side-reactions.

Also the reaction temperature needs to be controlled, and in general it should be kept below 60 °C.

Preferably the reaction temperature is between 0 °C and 50 °C but most preferable and convenient is room temperature. The reaction time varies depending on the other reaction parameters. Since the reaction course may be followed by TLC or preferably HPLC procedures, the skilled man is capable of controlling the reaction conditions and deciding when the reaction is to be considered as complete.

A preferred embodiment of this process is represented by a process as above described wherein the polar organic solvent is a mixture of dimethylformamide and dimethylsulfoxide, the strong alkali is aqueous concentrated potassium hydroxide and the reaction temperature is room temperature. Preferably, the proportion between dimethylformamide (DMF) and dimethylsulfoxide (DMSO) is from 4:1 to 3:2 (v/v), while the preferred concentration of the aqueous potassium hydroxide is between 85 and 90% (w/w).

Another preferred embodiment of this process is represented by a process as above described wherein the strong alkali is an alkali metal alkoxide as above described and the polar organic solvent is the alkanol corresponding to said alkoxide optionally in the presence of a polar aprotic solvent as defined above which is preferably dimethylformamide. The preferred mixture of strong alkali/polar organic solvent is in this case 1-10% methanolic sodium methoxide in dimethylformamide.

When the starting material is antibiotic L 17046 i.e. a starting material of the above formula II wherein A and M represent hydrogen atoms and OB is a sugar moiety as above defined, the corresponding deglycosylated compound is obtained under mild basic conditions such as for instance a molar excess (50-100 times excess) of an alkali metal alkoxide in the presence of the corresponding alkanol at room temperature for about 24-72 h followed by mild acid treatment, e.g. with an aqueous solution of a mineral acid, typically 0.5-1N hydrochloric acid.

In addition, the sugar moiety of a compound of formula I may be selectively removed to transform it into another compound of formula I.

A compound of formula I wherein OA and OM represent a sugar moiety as above defined, can be transformed into the corresponding compound wherein OM is as above and A is hydrogen by means of controlled acid hydrolysis in a strong concentrated aqueous organic acid. The concentrated organic acid in this case is preferably aqueous trifluoroacetic acid at a concentration between 75% and 95%, and the reaction temperature is preferably between 10° and 50°C. The preferred hydrolysis conditions are represented by about 90% trifluoroacetic acid at room temperature. The reaction time varies depending on the other specific reaction parameters but, in any case, the reaction may be monitored by TLC or preferably HPLC techniques. An analogous selective hydrolysis is reported in European Patent Application Publication No. 146822.

According to another embodiment of the present invention, a compound of formula I wherein OA and OM represent sugar moieties as defined above or a compound of formula I wherein A represents hydrogen and OM represents the above defined sugar moiety, may be transformed into the corresponding compound of formula I wherein A and M represent hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly miscible with water, phenylsubstituted lower alkanols wherein the phenyl moiety may optionally carry $(C_1-C_4)$alkyl, $(C_1-C_4)$alkoxy or halo groups which at the reaction temperature are liquids slightly miscible with water, and beta-polyhalogenated lower alkanols, which at the reaction temperature are liquids; in the presence of a strong acid, compatible with the solvent, selected from strong mineral acids, strong organic acids and strong acid cation exchange resins in the hydrogen form and at a temperature between 20°C and 100°C.

Alternatively, the selective hydrolysis can be conducted in an aprotic solvent such as a lower alkyl ether or polyether, e.g. dimethoxyethane, in the presence of a strong mineral acid, such as a hydrogen halide, e.g. hydrogen chloride or bromide. Conveniently, the preferred temperature is, in this case, room temperature.

In this case, the preferred hydrolysis conditions are represented by the use of a mineral acid, such as hydrochloric acid, in an haloalkanol such as trifluoroethanol, at a temperature between 65°C and 85°C.

Analogous selective hydrolysis conditions on a similar substrate are described in European Patent Application Publication No. 146053.

Preferred addition salts of the compounds of this invention are the pharmaceutically acceptable acid and/or base addition salts.

With the term "pharmaceutically acceptable acid and/or base addition salts", those salts with acids and/or bases are intended which from biological, manufacturing or formulation standpoint are suitable for use in the pharmaceutical practice as well as in animal growth promotion.

Representative and suitable acid addition salts of the compounds of formula I include those salts formed by standard reaction with both organic and inorganic strong acids such as, for example, hydrochloric, hydrobromic, sulfuric, phosphoric, trifluoroacetic, trichloroacetic, methanesulfonic, benzenesulfonic, picric, benzoic acid and the like.

Representative examples of salt forming bases may include: alkali metal or alkaline-earth metal hydroxide, such as sodium, potassium or calcium hydroxide; ammonia and inorganic aliphatic, alicyclic or aromatic amines such as methylamine, dimethylamine, trimethylamine, diethylamine, pyridine, piperidine and picoline.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present

invention.

For instance, a compound of formula I can be transformed into the corresponding acid or base addition-salt by dissolving the non-salt form in an aqueous solvent and adding a slight molar excess of the selected acid or base. The resulting solution or suspension is then lyophilized to recover the desired salt. Instead of lyophilizing, in some instances, it is possible to recover the final salt by extraction with an organic solvent, concentration to a small volume of the separated organic phase and finally precipitation by adding a non-solvent.

In case the final salt is insoluble in an organic solvent where the non-salt form is soluble it is recovered by filtration from the organic solution of the non-salt form after adding the stoichiometric amount or a slight molar excess of the selected acid or base.

The non-salt form can be prepared from a corresponding acid or base salt dissolved in an aqueous solvent which is then neutralized to free the non-salt form. This is then recovered for instance by extraction with an organic solvent or is transformed into another base or acid addition salt by adding the selected acid or base and working up as above.

When following the neutralization, desalting is necessary, a common desalting procedure may be employed.

For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique. After formation and isolation, the salt form of a compound of formula I can be transformed into the corresponding non-salt or into a pharmaceutically acceptable salt.

However, in view of the similarity of the properties of the compounds of formula I and their salts, what is said in the present application when dealing with the biological activities of the compounds of formula I applies also to their pharmaceutically acceptable salts, and vice versa.

The compounds of the invention are useful as semi-synthetic antibacterial agents active mainly against gram-positive bacteria.

Some physicochemical and biological data of representative compounds of the invention are reported in the following tables:

TABLE I

| Compound | OA | OM |
|---|---|---|
| 1(a-e) | $GNHCOR_{1-5}$ | -M |
| 1a | $GNHCOR_1$ | -M |
| 1b | $GNHCOR_2$ | -M |
| 1c | $GNHCOR_3$ | -M |
| 1d | $GNHCOR_4$ | -M |
| 1e | $GNHCOR_5$ | -M |
| 2 or 1(b-e) | $GNHCOR_{2-5}$ | -M |
| 3 | OH | -M |
| 4 | OH | -OH |

Note:

$-GNHCOR_{1-5}$ = N[($C_{10}$-$C_{11}$)aliphatic acyl]-beta-D-2-deoxy-2-aminoglucopyranosyl wherein ($C_{10}$-$C_{11}$)aliphatic acyl represents $R_1$, $R_2$, $R_3$, $R_4$ and $R_5$ groups as defined below:
$R_1$= (Z)-4-decenoyl, $R_2$= 8-methylnonanoyl, $R_3$= decanoyl, $R_4$= 8-methyldecanoyl and $R_5$= 9-methyldecanoyl

-M = alpha-D-mannopyranosyl

HPLC Analysis

The following table reports the $t_R$ of representative examples of the compounds of the invention.

The assays were run with a VARIAN model 5000 LC pump equipped with a 20 µl loop injector Rheodyne Model 7125 and a PERKIN-ELMER LC 15 UV detector at 254 nm.

Sample solution: 1 mg of test compound in 1 ml of 0.01 N $HCl/CH_3CN$, 3:7 (v/v)

Columns: pre-column (5 cm) packed with Perisorb RP-8 (silanized silica gel bearing C-8 aliphatic

chains; 30 µm; Merck Co.) followed by a stainless steel (250 x 4 mm) column Hibar RT 250-4 (Merck); pre-packed with Li-Chrosorb RP-8 (silanized silica gel bearing C-8 aliphatic chains; 10 µm; Merck Co.)

Eluents: A = 0.2% aqueous $HCO_2NH_4$

B = 100% $CH_3CN$

Elution conditions: linear gradient of B in A from 15 to 35% in 30 min.

Flow rate: 2 ml/min

TABLE II

| Compound | $t_R$ |
|---|---|
| 1a | 15.9 |
| 1b | 17.7 |
| 1c | 18.2 |
| 1d | 20.7 |
| 1e | 21.4 |
| 3 | 7.8 |
| 4 | 10.1 |
| teicoplanin $A_2$ component 1 | 14.2 |
| teicoplanin $A_2$ component 2 | 16 |
| teicoplanin $A_2$ component 3 | 15.5 |
| teicoplanin $A_2$ component 4 | 18.9 |
| teicoplanin $A_2$ component 5 | 19.8 |
| antibiotic L 17054 | 6.8 |

The following table (Table III) lists the analytical results, salt form, $(M + H)^+$ peak in the fast atom bombardment (FAB) mass spectra, and potentiometric analysis results.

Analytical results for C, H, N, and Cl were within ± 0.4% of the theoretical values. The weight loss (solvent content), determined by thermogravimetric analysis (TGA), was always about 6-7%. The inorganic residue, determined in oxygen atmosphere at 900 °C, was always <0.3%. The analytical results refer to the salt form reported in the table.

FAB-MS positive ion spectra were recorded on a Kratos MS-50 instrument fitted with a standard FAB source and high field magnet. The sample was dispersed in a mixture thioglycerol:diglycerol 1:1 (v/v) and bombarded with a 6-9 keV beam of Xe atoms.

Acid-base titration ($pK_{MCS}$) were carried out in 2-methoxyethanol (Methyl Cellosolve $^R$):water 4:1 (v/v) solution, with 0.01 NaOH after addition of suitable amounts of 0.01N HCl. The equivalent weights (EW) given are corrected for the solvent content and the inorganic residue.

TABLE III

Physico-chemical and analytical data of representative compounds of the invention.

| COMPOUND | FORMULA (MW) | SALT FORM | FAB-MS $(M + H)^+$ | ACID-BASE TITRATION $pK_{mcs}$ (EW) |
|---|---|---|---|---|
| 1(a-e) | | HCl | | |
| 1a | | | | |
| 1b | (1659) | HCl | 1659 | |
| 1c | | | | |
| 1d | (1673) | HCl | 1673 | |
| 1e | | | | |
| 2 | | HCl | | 5.00, 6.95 (840) |
| 3 | $C_{64}H_{55}Cl_2N_7O_{22}$ (1345) | $CF_3COOH$ | 1346 | 5.05, 6.90 (695) |
| 4 | $C_{58}H_{45}Cl_2N_7O_{17}$ (1183) | HCl | 1183 | 5.15, 7.05 (594) |

## TABLE IV

The I.R. most significant bands ($cm^{-1}$) of some compounds (as the corresponding hydrochlorides) of the invention recorded with a Perkin-Elmer 580 spectrometer in nujol mull are reported below:

| COMPOUND | |
|---|---|
| 1,2 | 3700-3100 ( $\nu$NH, and $\nu$OH glycosidic and phenolic); 1730 ($\nu$C=O, COOH); 1645 (amide I); 1505 (amide II) |
| 3 | 3700-3100 ( $\nu$NH, and $\nu$OH glycosidic and phenolic); 1725 ($\nu$C=O, COOH); 1645 (amide I); 1515 (amide II) |
| 4 | 3700-3100 ( $\nu$NH, and $\nu$OH phenolic); 1730 ($\nu$C=O, COOH); 1650 (amide I); 1505 (amide II) |

TABLE V

UV-Vis data of some representative compounds of the invention recorded with a Unicam SP 500 spectrophotometer are reported below (Lambda max, nm):

| Compound | 0.1N HCl | Phosphate buffer pH 7.4 | 0.1N KOH |
|---|---|---|---|
| 1,2 | 280 | 280 | 295 |
| 3 | 280 | 280 | 295 |
| 4 | 280 | 280 | 296 |

TABLE VI

| Significant $^{1}H$-NMR assignments of some representative compounds of the invention recorded in DMSO-$d_6$ at 30 °C with a Bruker WH-270 cryospectrometer, using tetramethylsilane (TMS) as the internal standard (delta = 0.00 ppm): | |
|---|---|
| compound 2 | 0.82, 1.15, 1.42, 2.01 (acyl chains); 3.00, 3.25 ($H'_{34}$, $H_{34}$); 3.51 (mannose); 5.10 ($H_{35}$); 4.05-5.60 (peptidic CH's); 6.23-7.95 (aromatic protons) |
| Compound 3 | 2.96, 3.26 ($H'_{34}$, $H_{34}$); 3.48 (mannose); 5.12 ($H_{35}$); 3.97-5.62 (peptidic CH's); 6.15-7.98 (aromatic protons) |
| Compound 4 | 2.99, 3.25 ($H'_{34}$, $H_{34}$); 5.11 ($H_{35}$); 3.98-5.60 (peptidic CH's); 6.13-7.92 (aromatic protons) |

The antibacterial activity of the compounds of the invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37 °C. The results (MIC) of the antibacterial testing of representative compounds of formula I are summarized in the following Table VII together with data on the in vivo efficacy. The $ED_{50}$ values (mg/kg) of the compounds are obtained as determined in mice

experimentally infected with S. pyogenes L49 according to the procedure described by V. Arioli et al., Journal of Antibiotics 29, 511 (1976).

TABLE VII

| TEST ORGANISM | M.I.C. (µg/ml) COMPOUND 1(a-e) | 2 | 3 | 4 |
|---|---|---|---|---|
| Staph. aureus L165 | 0.125 | 0.125 | 0.25 | 0.25 |
| Staph. epidermidis L147 ATCC 12228 (coagulase negative) | 0.125 | 0.063 | 0.25 | 0.125 |
| Staph. haemolyticus L602 (coagulase negative) | 1 | 1 | 4 | 1 |
| Strep. pyogenes L49 C203 | 0.063 | 0.063 | 1 | 1 |
| Strep. pneumoniae L44 UC41 | 0.032 | 0.063 | 1 | 2 |
| Strep. faecalis L149 ATCC 7080 | 0.25 | 0.125 | 1 | 2 |
| Escherichia coli L47 SKF 12140 | >128 | >128 | >128 | >128 |
| $ED_{50}$ (mg/kg) s.c. | 0.98 | 0.94 | 6.6 | n.d. |
| os | 280 | 270 | >300 | n.d. |

In view of the above reported antimicrobial activity, the compounds of the present invention can effectively be employed as the active ingredients of antimicrobial preparations used in human and

veterinary medicine for the prevention and treatment of infectious diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints.

For medication of the eyes or ears, the preparation may be presented in liquid or semi-liquid form. Topical applications may be formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylene glycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compounds of the invention are generally effective at a dosage comprised between about 0.5 and about 30 mg of active ingredient per kg of body weight, preferably divided in 2 to 4 administrations per day. Particularly desirable compositions are those prepared in the form of dosage units containing from about 20 to about 300 mg per unit.

Representative examples of preparation of pharmaceutical compositions are as follows:

A parenteral solution is prepared with 100 mg of compound 1b dissolved in 2 ml of sterile water for injection.

A parenteral solution is prepared with 250 mg of compound 2 dissolved in 3 ml of sterile water for injection.

A topical ointment is prepared with 200 mg of compound 3

3.6 g of polyethylene glycol 400 U.S.P.

6.2 g of polyethylene glycol 4000 U.S.P

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and incorporating the premix into the complete ration.

Alternatively, an intermediate concentrate or feed supplement containing the active ingredient can be blended into the feed.

The way in which such feed premixes and complete rations can be prepared and administered is described in reference books (such as "Applied Animal Nutrition", W.H. Freedman and Co., S. Francisco, USA, 1969 or "Livestock Feeds and Feeding", O and B Books, Corvallis Oregon, USA, 1977) which are incorporated herein by reference.

Some physico-chemical data of intermediates of formula III are reported in the following table.

TABLE VIII

| Compound | OA | OM | 36,37-Bond |
|---|---|---|---|
| I | -GNHCOR | -M | transoid |
| II | OH | -M | transoid |
| III | OH | OH | transoid |

Note:

-GNHCOR = N/(C$_{10}$-C$_{11}$)aliphatic acyl/-beta-D-2-deoxy-2-aminoglucopyranosyl as above defined for the teicoplanin $A_2$ components

-M = alpha-D-mannopyranosyl

The following table (Table IX) lists the analytical results, salt form, $(M + H)^+$ peak in the fast atom bombardment (FAB) mass spectra, and potentiometric and HPLC analysis results.

Analytical results for C, H, N, and Cl were within ± 0.4% of the theoretical values. The weight loss (solvent content), determined by thermogravimetric analysis (TGA), was always about 6-7%. The inorganic residue, determined in oxygen atmosphere at 900 °C,was always <0.3%. The analytical results refer to the salt form reported in the table.

FAB-MS positive ion spectra were recorded on a Kratos MS-50 instrument fitted with a standard FAB source and high field magnet. The sample was dispersed in a mixture thioglycerol:diglycerol 1:1 (v/v) and bombarded with a 6-9 keV beam of Xe atoms.

Acid-base titrations ($pK_{MCS}$) were carried out in 2-methoxyethanol (Methyl Cellosolve®):water 4:1 (v/v) solution, with 0.01M NaOH after addition of suitable amounts of 0.01N HCl. The equivalent weights (EW) given are corrected for the solvent content and the inorganic residue.

HPLC was run with a Varian Mod. 5000 LC pump equipped with a 20 µl injector Rheodyne Mod. 7125 and a UV detector at 254 nm.

Columns: a pre-column (5 cm) packed with Perisorb RP-8 (30 µm) (silanized silica gel bearing C-8 aliphatic chains; Merck Co.) followed by a stainless steel (250 x 4 mm) column Hibar RT 250-4 (Merck); pre-packed with Li-Chrosorb RP-8 (silanized silica gel bearing C-8 aliphatic chains; 10 µm; Merck Co.).

Chromatographic conditions: eluent A, 0.2% aq. $HCO_2NH_4$; eluent B, 100% $CH_3CN$; linear gradient from 15 to 35% of B in A in 30 min at the flow rate of 2 ml/min; injection 20 µl. The reactions were monitored by

injecting samples of the solutions diluted enough to obtain a final concentration of about 1 mg/ml. Final products were checked by injecting solutions (20 µl) of 10 mg of each product in 10 ml of a mixture $CH_3CN$:0.2% aq. $HCO_2NH_4$ (or 0.1N HCl for Intermediate III) 1:1 (v/v).

TABLE IX

Physico-chemical and analytical data of representative compounds of formula III

| COMPOUND | FORMULA (MW) | SALT FORM | FAB-MS $(M + H)^+$ | *POTENTIOMETRIC $pK_{mcs}$ (EW) | HPLC ($t_R$, min) |
|---|---|---|---|---|---|
| I | - | HCl | n.d. | 5.0 (COOH)<br>6.9 ($NH_2$)<br>(1870) | (component 1) 15.7<br>(component 2) 17.5<br>(component 3) 18.1<br>(component 4) 20.4<br>(component 5) 21.2 |
| II | $C_{64}H_{53}Cl_2N_7O_{22}$<br>(1343) | HCl | 1342 | 4.8 (COOH)<br>6.9 ($NH_2$)<br>(1440) | 7.74 |
| III | $C_{58}H_{43}Cl_2N_7O_{22}$<br>(1181) | HCl | 1180 | n.d. | 10.06 |

* The data for teicoplanin $A_2$ were: $pK_{mcs}$ 5.0 (COOH), 7.1 ($NH_2$) with an average EW 1930;
The data for deglucoteicoplanin were: $pK_{mcs}$ 4.9 (COOH), 6.9 ($NH_2$) with EW 1407;
**n.d. = Not determined.**

TABLE X

Some significant $^1$H-NMR assignments for the compounds I to III in comparison with deglucoteicoplanin (delta = ppm; m - multiplicity) $^1$H-NMR spectra were recorded in DMSO-$d_6$ at 30°C with a Bruker WH-270 cryospectrometer, using tetramethylsilane (TMS) as the internal reference (delta = 0.00 ppm).

| PROTONS | COMPOUNDS | | | | | | | |
|---|---|---|---|---|---|---|---|---|
| | I | | II | | III | | DEGLUCOTEICOPLANIN | |
| $C_{15}$-H | 4.69 | (s) | 4.66 | (s) | 4.67 | (s) | 4.62 | (s) |
| $C_{18}$-H | 4.93 | (ddd) | 4.94 | (ddd) | 4.93 | (ddd) | 4.97 | (d) |
| $C_3$-H | 5.33 | (d) | 5.29 | (d) | 5.28 | (d) | 5.34 | (d) |
| $C_{50a}$-H | 5.67 | (d) | 5.65 | (d) | 5.55 | (d) | 5.67 | (d) |
| $C_{48}$-H | 4.53 | (d) | 4.57 | (d) | 4.58 | (d) | 4.34 | (d) |
| $C_{35}$-H | - | | - | | - | | 4.12 | (dd) |
| $C_{38}$-H | 4.13 | (d) | 4.15 | (d) | 4.00 | (d) | 4.39 | (d) |
| $C_{19}$-H | 2.86 | (dd) | 2.86 | (dd) | 2.82 | (dd) | 2.85 | (dd) |

Continued..

TABLE X

| PROTONS | COMPOUNDS I | | II | | III | | DEGLUCOTEICOPLANIN | |
|---|---|---|---|---|---|---|---|---|
| $C_{34}$-H | 4.13 | (d) | 4.15 | (d) | 4.16 | (d) | 5.10 | (d) |
| $C_{34}$-H | 4.76 | (d) | 4.83 | (d) | 4.93 | (d) | - | |
| $C_{26}$-H | 5.59 | (s) | 5.58 | (s) | 5.40 | (s) | 5.51 | (s) |
| $C_{27}$-H | 4.72 | (s) | 4.71 | (s) | 4.68 | (s) | 5.11 | (s) |
| CH of MANNOSE | 3.48 | (m) | 3.48 | (m) | - | | - | |
| ANOMERIC H of MANNOSE | 5.48 | (s) | 5.44 | (s) | - | | - | |
| $N_{52}$-H | - | | - | | - | | 6.72 | (d) |
| $N_{37}$-H | n.d. | | n.d. | | n.d. | | 8.36 | (d) |

n.d.: not determined

multiplicity: s = singlet; d = doublet; dd = doublet of doublet; ddd = doublet of doublet of doublet; m = multiplet

TABLE XI

UV Spectral data (Lambda max, nm) of the compounds I to III (UV spectra were run on a Unicam SP 800 Spectrophotometer).

| SOLVENT | COMPOUND I | II | III |
|---|---|---|---|
| $CH_3OH$ | n.d. | n.d. | 282 (26870) |
| 0.1N HCl | 279 (14980) | 279 (15850) | 280 (17310) |
| 0.1N NaOH | 296 (18780) | 296 (23140) | 296 (24490) |
| $H_2O$ buffer pH 7.4 | 279 (17650) | 279 (17650) | 280 (19000) |

n.d. = not determined

TABLE XII

IR Spectral data ($cm^{-1}$) of the compounds I to III in comparison with deglucoteicoplanin (recorded on a Perkin Elmer 580 spectrometer in nujol mull).

| COMPOUND | NH, and PHENOLIC OH | AMIDE I | $\nu$ C=O (COOH) (or $COO^-$) | $\delta$ $NH_3^+$ | AMIDE II | PHENOLIC $\delta$ OH and $\nu$ C-O | BENDING VIBRATION OF MANNOSE |
|---|---|---|---|---|---|---|---|
| I | 3280 (broad) | 1645 | 1610 | 1590 | 1510 | 1230, 1060, 1025-1010 | 970 |
| II | 3240 (broad) | 1650 | 1725 (COOH) | 1590 | 1515 | 1225, 1060 1005 | 970 |
| III | 3300 (broad) | 1655 | 1615 | 1590 | 1515 | 1230, 1060 1010 | - |
| DEGLUCO-TEICOPLANIN | 3300 | 1655 | 1610 | 1590 | 1515 | 1230, 1140, 1060, 1005 | - |

The following Preparations and Examples are intended to further illustrate the invention and must not be interpreted as limiting it.

Preparation 1:

Preparation of 42-alpha-D-mannosyl-56-N-acyl-beta-D-glucosaminyl-35,52-didehydro-34-deoxy-teicoplanin aglycon (Intermediate I)

a) A solution of 50 g (0.75 mol) of commercial 85% KOH in 1 liter of $CH_3OH$ is added dropwise at room temperature to a stirred solution of 23 g (12 mmol) of teicoplanin (containing about 15% w/w of water) in 2.5 1 of mixture DMF/DMSO 3:2 (v/v). The resulting suspension is stirred at room temperature (the reaction vessel being sealed with a valve containing soda lime) for 24 h. After standing 48 h at 10 °C, 1 liter of methanol is added and the resulting brown solution is stirred at room temperature for 2 h. By adding 1 liter of ether a solid separates which is collected and suspended in 1 liter of methanol. The insoluble is collected, washed with 1 liter of ether and dried in vacuum at room temperature overnight over $P_2O_5$, yielding 21 g of the crude compound of the title in the form of the corresponding potassium salt.

7 g of this crude potassium salt is dissolved in 400 ml of a mixture $CH_3CN/H_2O$ 1:2 (v/v) and the resulting cloudy solution is brought to pH 2.8 with glacial acetic acid, then 25 g of silanized silica gel (Silicagel 60, Merck Co.; 0.06-0.2 mm) and 500 ml of butanol are added. The solvents are completely evaporated under reduced pressure and the residue is placed on top of a column containing 1.5 kg of the same silanized silica gel in 0.01M aqueous $NH_4H_2PO_4$. The column is developed with a linear gradient from 10% to 70% $CH_3CN$ in 0.5% aq. acetic acid in 30 h at a rate of 300 ml/h. Fractions of 25 ml each are collected and checked by HPLC. Those containing the five pure components of the complex of the title are pooled, butanol is added and the mixture is concentrated until both $CH_3CN$ and $H_2O$ are completely removed. Then 5 ml of 1N HCl is added and the solution is concentrated to a small volume. Ethyl acetate is then added to precipitate a solid which is collected by filtration, washed with ether and dried in vacuo at room temperature overnight, yielding 5.04 g of pure hydrochloride of the compound of the title.

b) Preparation of the internal salt

A solution of 1.7 g ( 1 mmol) of this hydrochloride in 200 ml of a mixture of $H_2O/CH_3CN$ 2:1 (v/v) is adjusted to pH 6 with 0.1N NaOH. After adding 80 ml of butanol, the mixture is concentrated to a final volume of about 40 ml. A solid separates which is collected by filtration, washed subsequently with 50 ml of water and a mixture of acetone/ethyl ether 1:2 (v/v) and dried in vacuum at room temperature (over $P_2O_5$) for 48 h, yielding 1.4 g of the title compound, as the internal salt.

Preparation 2:

Preparation of 42-alpha-D-mannosyl-35,52-didehydro-34-deoxy-teicoplanin aglycon (Intermediate II)

a) From Antibiotic L 17054

A solution of 1.8 g (27 mmol) of commercial 85% KOH in 200 ml of methanol is added at room temperature to a stirred solution of 1.1 g (0.7 mmol) of antibiotic L 17054 in 300 ml of a mixture DMF/DMSO 3:2 (v/v). The suspension which forms is stirred at room temperature for 30 h, then 800 ml of ethyl ether is added. The precipitate is collected, washed with ether and dried in vacuo at room temperature overnight, yielding 1.2 g of crude compound of the title as the potassium salt, which is then purified by column chromatography on a column packed with 750 g of silanized silica gel under the conditions described above for Intermediate I, but eluting with a linear gradient from 5% to 35% acetonitrile in water in 24 h at a rate of 250 ml/h. Fractions containing the pure compound of the title are pooled and worked up as described in the foregoing Preparation. After removing both acetonitrile and water, 1 ml of 1N HCl is added to the resulting butanolic solution which is concentrated to a final volume of about 40 ml. By adding ether a solid separates which is collected by filtration, washed with ether and dried in vacuo overnight at 35 °C, yielding 0.6 g of the hydrochloride of the compound of the title.

b) From Intermediate I

i) A solution of 5 g (3 mmol) of Intermediate I as obtained by following the procedure of the foregoing Preparation 1 in 60 ml of 90% aqueous trifluoroacetic acid (TFA) is stirred for 10 min at 5 °C and 90 min at room temperature. Then 240 ml of ethyl ether is added and the precipitate which separates is collected and redissolved in 200 ml of methanol. The insoluble is removed by filtration and the filtrate is concentrated to a small volume under reduced pressure. By adding ether, a solid separates which is collected, washed with ether and dried in vacuo at 35 °C overnight, yielding 3.96 g of pure trifluoroacetate of the compound of the title.

ii) A solution of 6 g (3 mmol) of crude (85% titre by HPLC expressed as the percentage of the areas of peaks; the impurities were due to undefined by-products) Intermediate I potassium salt in 100 ml of 90% aqueous TFA is stirred 15 min at 0 °C and 2 h at room temperature. The solvents are completely evaporated at room temperature under reduced pressure. The oily residue is dissolved in 150 ml of a mixture of $H_2O$:$CH_3CN$ 1:1 (v/v) and the resulting solution is diluted with 300 ml of water and loaded at the top of a column containing 750 g of silanized silica gel in the mixture 0.5% aq. $HCO_2NH_4$:$CH_3CN$ 90:10 (v/v). The column is first washed with 500 ml of the mixture $H_2O$:$CH_3CN$ 85:15 (v/v) and then developed with a linear gradient from 15 to 30% of acetonitrile in 0.001N HCl in 24 h at the rate of 250 ml/h, while collecting 25 ml fractions. Those containing the pure compound of the title were pooled. Some crystalline product present is collected, washed with a mixture acetonitrile/ethyl ether 1:2 (v/v) and dried in vacuum at room temperature overnight (over $P_2O_5$), yielding 0.85 g of pure compound of the title, as the internal salt. The mother liquors are concentrated after adding enough butanol to obtain a final dry butanolic suspension of about 200 ml. After adding 3 ml of 1N HCl, a clear solution forms which is concentrated to a small volume under reduced pressure. By adding ethyl ether, a solid separates which is collected by filtration, washed with ether and dried in vacuo at room temperature overnight (over KOH pellets), obtaining 2.3 g of pure hydrochloride of the compound of the title.

Preparation 3:

Preparation of 35,52-didehydro-34-deoxy-teicoplanin aglycon (Intermediate III)

a) From Intermediate I

Dry HCl is slowly bubbled into a stirred suspension of 0.6 g of Intermediate I (see Preparation 1) in 50 ml of dimethoxyethane (DME), while maintaining the internal temperature at 15-20 °C. A clear solution forms in a few hours which is then evaporated to dryness under reduced pressure. The residue is dissolved in 100 ml of a mixture $H_2O$:$CH_3CN$ 75:25 (v/v) and the resulting solution is loaded at the top of a column of 100 g of silanized silica gel in $H_2O$. The column is developed with a linear gradient from 25 to 60% $CH_3CN$ in 0.001N HCl in 20 h at the rate of 150 ml/h, while collecting 15 ml fractions. Those containing pure Intermediate III are pooled and concentrated after adding enough butanol to obtain a final dry butanolic solution of about 100 ml. By adding 300 ml of dry hydrochloric ethyl ether, a solid separates which is collected, washed with ether and dried in vacuo at room temperature for 48 h (over KOH pellets), obtaining 0.35 g of the title compound, as the hydrochloride.

b) From Intermediate II

To a stirred suspension of 3 g of Intermediate II (see Preparation 2) in 100 ml of dimethoxyethane (DME) 25 ml of 96-98% $H_2SO_4$ is added dropwise while cooling to 0 °C. The solution which forms is stirred at room temperature for 5 h, then 250 ml of ether is added and the solid which separates is collected, washed with ether and dried in vacuo at room temperature overnight, yielding 2.5 g of the title compound, as the sulphate.

Example 1: Preparation of Compound 1(a-e)

(Method A1)

To a stirred solution of 10 g (about 5 mmol) of teicoplanin $A_2$ in 1.5 liter of a mixture DMF:$CH_3OH$, 2:1 (v/v), 100 g of sodium borohydride (pellets of about 0.4 g each, Aldrich) is added at 5 °C-14 °C. After stirring at 10 °C-15 °C for 6 h and at 20 °C-25 °C for 120 h, the reaction mixture is poured into a solution of 160 ml of glacial acetic acid in 3 liters of methanol, while cooling at 5 °C-10 °C. The resulting solution is concentrated at 45 °C under reduced pressure to a volume of about 150 ml and the precipitate is filtered off. The filtered solution is then evaporated to dryness at 50 °C under reduced pressure. The residue (about 19 g) which contains inorganic salts and about 50% of a mixture of the compound of the title and the corresponding 35,52-didehydro derivative (in a 6:4 ratio as results from U.V. differential titration and $^1$H-N.M.R. spectroscopy) is redissolved in a cold (0 °C-5 °C) solution of 37% hydrochloric acid (126 ml) in dimethylformamide (400 ml). After adding Zn powder (33 g) the resulting mixture is stirred at 20 °C-22 °C for 1 h. During this period, the 35,52-didehydro derivative is completely transformed into a more lipophilic derivative, while the compound of the title remains unmodified. The insoluble is then filtered off and 1.5 liter of water is added to the filtered solution. After extraction with 1.5 liter of n-butanol, the organic layer is washed with water (2 x 500 ml), then it is brought to pH 6 with 1N sodium hydroxide and concentrated to a

small volume (about 100 ml) at 45 °C under reduced pressure. The concentrated cloudy butanolic solution is poured into 1 liter of a mixture $CH_3CN:H_2O$ 2:8 (v/v) under vigorous stirring and the resulting solution is adjusted to pH 3 with 0.1N hydrochloric acid and loaded on a column of 1.4 kg of silanized silica gel (0.063-0.2 mm; Merck) in the same solvent mixture [$CH_3CN:H_2O$ 2:8 (v/v)]. The column is then developed with a linear gradient from 20% to 70% of acetonitrile in 0.001N hydrochloric acid in 20 h at the flow rate of about 400 ml/h, while collecting 20 ml fractions which are monitored by HPLC. Those containing pure components 1 to 5, or mixtures thereof, of the title compound are pooled and two volumes of n-butanol are added. The resulting mixture is concentrated to a small volume (about 100 ml) at 35 °C under reduced pressure and ethyl ether (300 ml) is added. A solid separates which is collected by filtration, washed with ethyl ether (200 ml) and dried at room temperature in vacuo overnight, yielding 0.79 g (about 8.5%) of compound 1(a-e), hydrochloride.

By collecting components 1a, 1b, 1c, 1d and 1e separately from the above reported reverse-phase column chromatography or by starting from any of components 1 to 5 of teicoplanin $A_2$ instead of the complex, the corresponding single components (i.e. compounds 1a, 1b, 1c, 1d or 1e) are obtained in a pure form.

Example 2: Preparation of Compound 2

a) From teicoplanin $A_2$ (Method A2)

To a stirred solution of 10 g (about 5 mmol) of teicoplanin $A_2$ in 200 ml of a mixture $DMF:CH_3OH$ 1:1 (v/v), 75 g of sodium borohydride (pellets of about 0.4 g each, Aldrich) is added at 0 °C-5 °C. The reaction mixture is stirred at 5 °C-10 °C for 3 h and at room temperature for 24 h, then it is poured into a solution of glacial acetic acid (150 ml) in $CH_3OH$ (1 l) with stirring while cooling to 10 °C-15 °C. The resulting solution is concentrated to a volume of about 200 ml at 45 °C under reduced pressure and the precipitate which forms is filtered off. After adding 100 ml of water, the filtrate (essentially containing the compound of the title, as results from HPLC analysis) is hydrogenated at room temperature and pressure for 30 min in the presence of 5 g of 5% Pd/C, (about 200 ml of $H_2$ absorbed). Then, 5 g of the same catalyst is added and the hydrogenation is continued under the above conditions over a period of about 40 min, (additional 220 ml $H_2$ absorbed). The catalyst is filtered off through a panel of Celite BDH-545 filter-aid (20 g) and the filtrate is concentrated at 50 °C to a small volume (about 50 ml) under reduced pressure. On adding ethyl acetate (450 ml), a solid separates which is collected, washed with ether (200 ml) and redissolved in 200 ml of a mixture $CH_3CN:H_2O$ 1:1 (v/v). After adding 50 g of silanized silica gel (0.063-0.2 mm; Merck) and 800 ml of water under vigorous stirring, the resulting suspension is loaded at the top of a column of 1.4 kg of the same silica gel in water. The column is developed with a linear gradient from 10% of $CH_3CN$ in 0.001N HCl to 60% of $CH_3CN$ in 0.01N HCl in 10 h at the rate of 600 ml/h, while collecting 25 ml fractions which are analyzed by HPLC. Those fractions containing the pure compound of the title are pooled and concentrated under reduced pressure at 25 °C after adding enough n-butanol to obtain a final dry butanolic cloudy solution of about 60 ml. On adding ethyl ether (about 240 ml) a solid separates which is collected by filtration, washed with 100 ml of ether and dried in vacuo at 40 °C overnight, yielding 0.76 g of the compound of the title as the hydrochloride. Other crops of compound 2 are obtained by pooling and working up substantially as above those fractions which contain it in less pure form.

By collecting components 1b, 1c, 1d and 1e separately from the above reverse phase column chromatography and working up as described above, single components 1b, 1c, 1d and 1e are obtained. By starting from any of the components of teicoplanin $A_2$ the corresponding pure derivative is obtained with the exception that, under these reaction conditions, teicoplanin $A_2$ component 1 always yields compound 1c.

b) From Intermediate I (Method B)

A solution of 3.4 g (about 2 mmol) of Intermediate I (see Preparation 1) in 300 ml of a mixture $CH_3CN$:0.04N HCl 7:3 (v/v) is hydrogenated at room temperature and pressure in the presence of 5% Pd/C (1.5 g). After 30 min, about 57 ml of $H_2$ is absorbed, then additional 2 g of the same catalyst is added and hydrogenation is continued under the above conditions. When about 70 ml of $H_2$ is absorbed, the catalyst is removed by filtration on Celite BDH-545 filter-aid. After adding 200 ml of water and 400 ml of n-butanol, the filtrate is concentrated at 25 °C under vacuo to evaporate most of the methanol. The organic layer is separated and concentrated at 25 °C under vacuo to a small volume (about 50 ml). On adding ethyl acetate (350 ml), a solid separates which is collected by filtration, washed with ether (100 ml) and dried at 40 °C in vacuo overnight, yielding 2.5 g of the title compound, as the hydrochloride.

Example 3: Preparation of Compound 3

a) From antibiotic L 17054 (Method A2)

The compound of the title as the corresponding hydrochloride, is obtained from antibiotic L 17054 substantially following the procedure described in Example 2a) with the same yield (about 8%). The only difference consists in the preparation of a second crop which is isolated as the internal salt as follows. The fractions (from reverse phase column chromatography as described in Example 2a) containing impure compound of the title are pooled and most of the acetonitrile is evaporated at 40 °C under reduced pressure. The resulting aqueous suspension is filtered and the filtrate is adjusted to about pH 1.2 with 37% hydrochloric acid. After standing at 6 °C overnight, a precipitate forms which is separated by filtration and redissolved in a mixture $CH_3CN$:$H_2O$ 1:1 (v/v), to a concentration of about 1 g/50 ml. The resulting solution is brought to about pH 5.9 with 1N NaOH and the acetonitrile is evaporated at 40 °C under vacuo. Pure title compound (about 14% yield) is then collected, as the internal salt, by filtration from the resulting aqueous suspension.

b) From Intermediate II (Method B)

Catalytic hydrogenation of Intermediate II (see Preparation 2) carried out essentially under the conditions described in Example 2b) gives the compound of the title as the hydrochloride with about the same yield (about 70%). In this case, the final product is recovered directly, after removing the catalyst, by evaporation of the solvents and trituration of the residue with acetone and ether.

c) From Compound 1 or Compound 2 (Method C)

A solution of 5 g (about 3 mmol) of compound 1 (see Example 1) or compound 2 (see Example 2) in 100 ml of 90% aqueous trifluoroacetic acid is stirred at room temperature over a period of about 90 min. Then, 300 ml of ether is added and the precipitate is collected and redissolved in 100 ml of methanol. The insoluble is filtered off and the filtrate is concentrated to a small volume (about 20 ml) at 35 °C under vacuo. On adding ether (80 ml), a solid separates which is collected by filtration, washed with ether (100 ml) and dried in vacuo at 40 °C overnight, yielding 3.8 g of pure title compound, as the trifluoroacetate.

Example 4: Preparation of Compound 4 (Method D)

a) From Compound 1 or 2

Dry HCl is slowly bubbled into a stirred suspension of 1.7 g (about 1 mmol) of compound 1 or compound 2 in 100 ml of 1,2-dimethoxyethane, while maintaining the temperature to 20 °C-25 °C. A clear solution forms within few minutes and after about 7 h, the solvent is evaporated at 50 °C under reduced pressure. The residue is purified by reverse-phase column chromatography as described above in Example 2b, thus obtaining 0.7 g of the title compound, as the hydrochloride.

b) From Compound 3

The reaction is carried out for 6 h under the same conditions as described above (see Example 4 a) but starting from 1.4 g (about 1 mmol) of compound 3 (see Example 3). Recovery of the crude product and its purification by reverse-phase column chromatography (as above), yields 0.9 g of the title compound as the hydrochloride.

**Claims**

**Claims for the following Contracting States : AT, BE, CH, DE, FR, GB, IT, LI, LU, NL, SE**

1. 34-De-(acetylaminoglucopyranosyl)-34-deoxy teicoplanin derivatives of the following formula I

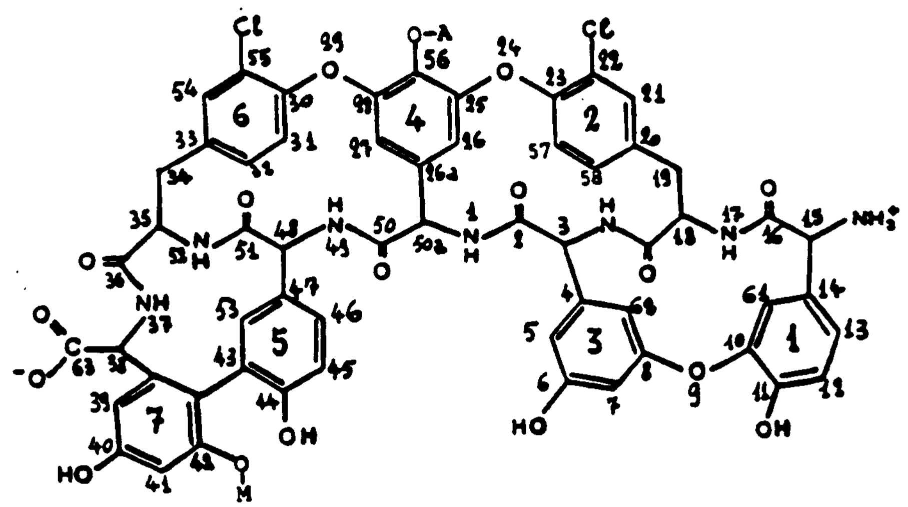

I

wherein

A represents hydrogen or OA represents N[($C_{10}$-$C_{11}$)aliphatic acyl]-beta-D-2-deoxy-2-aminoglucopyranosyl,

M represents hydrogen or OM represents alpha-D-mannopyranosyl, with the proviso that when M represents hydrogen also A must represent hydrogen,

and the addition salts thereof.

2. A compound of claim 1 wherein OA and OM represent the sugar moieties defined therein.

3. A compound of claim 1 wherein OA represents beta-D-2-deoxy-2-(8-methylnonanoyl)-aminoglucopyranosyl and OM represents alpha-D-mannopyranosyl.

4. A compound of claim 1 wherein A and M represent hydrogen.

5. A process for preparing a compound of claim 1, with the exclusion of a compound wherein OA represents beta-D-2-deoxy-2-((Z)-4-decenoyl)aminoglucopyranosyl, which comprises catalytically hydrogenating a compound of formula III:

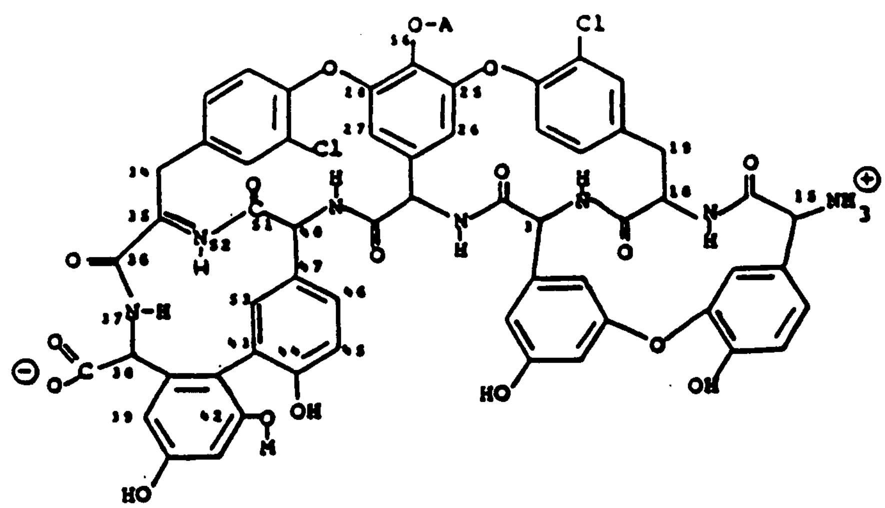

III

wherein,

A represents hydrogen or OA represents N[($C_{10}$-$C_{11}$)aliphatic acyl]-beta-D-2-deoxy-2-aminoglucopyranosyl,

M represents hydrogen or OM represents alpha-D-mannopyranosyl, with the proviso that when M represents hydrogen also A must represent hydrogen

and the addition salts thereof, in an aqueous alcoholic medium.

6. A process according to claim 5 wherein the reaction temperature is room temperature and the pressure is ambient pressure.

7. A process according to claim 5 wherein the hydrogenation catalyst is a transition metal or a transition metal derivative optionally on a suitable support.

8. A process according to claim 5 wherein the hydrogenation catalyst is selected from palladium on barium sulphate, palladium on charcoal, palladium on alumina, palladium on calcium carbonate, platinum, platinum oxide and rhodium on charcoal.

9. A process for preparing a compound of claim 1, which includes selectively removing the N-acetyl-beta-D-2-deoxy-2-aminoglucopyranosyl group from a compound of formula II:

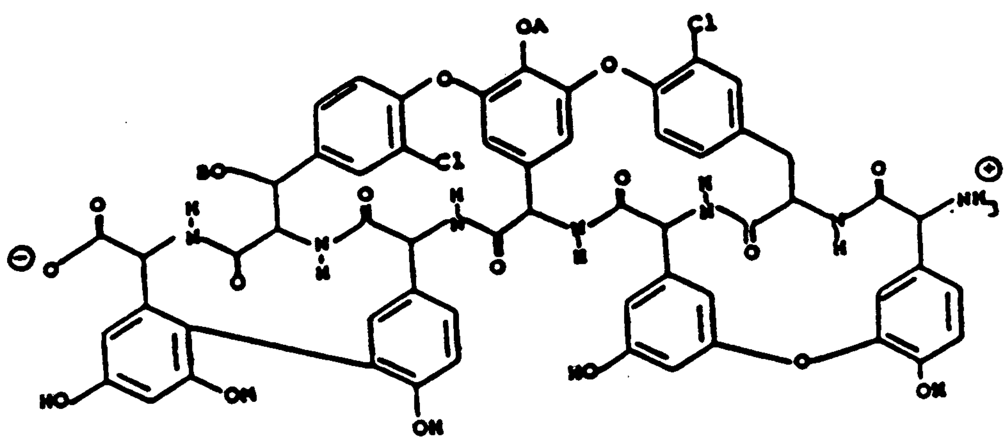

II

wherein A represents hydrogen or OA represents -N[$C_{10}$-$C_{11}$)aliphatic acyl]-beta-D-2-deoxy-2-aminoglucopyranosyl, B represents hydrogen or OB represents N-acetyl-beta-D-2-deoxy-2-aminoglucopyranosyl, M represents hydrogen or OM represents alpha-D-mannopyranosyl, with the proviso that B may represent hydrogen only when A and M are simultaneously hydrogen and M may represent hydrogen only when A is hydrogen, a salt thereof, or a mixture thereof, in any proportion, under conditions which do not affect the unsaturation on the ($C_{10}$-$C_{11}$)aliphatic acyl function.

10. A process according to claim 9 wherein the selective removal is conducted in the presence of a molar excess of an alkali metal or alkali-earth metal borohydride in a suitable medium.

11. A process according to claim 9 wherein the reaction medium is a mixture of a polar aprotic solvent and a polar protic solvent.

12. A process according to claim 11 wherein the reaction medium is a mixture of dimethylformamide and methanol, 1:1 (v/v).

13. A process according to claim 9 wherein the final product is treated with a mild reducing agent before separating it by known per se means from a more lipophilic by-product.

14. A process according to claim 13 wherein the mild reducing agent is represented by a molar excess of a metal capable of developing hydrogen from an aqueous acid solution or other redox agents having a similar standard reduction potential in the electrochemical series.

**15.** A process according to claim 13 wherein the mild reducing agent is zinc or tin in an aqueous mineral acid.

**16.** A process according to claim 13 wherein the reaction temperature is room temperature.

**17.** A compound of claim 1, 2, 3 or 4 for use as a medicine.

**18.** Use of a compound of claim 1, 2, 3, or 4 for preparing a medicament for antimicrobial use.

**19.** A pharmaceutical composition which contains a compound of claim 1, 2, 3 or 4 in admixture with a pharmaceutically acceptable carrier.

**Claims for the following Contracting States : ES, GR**

**1.** A process for preparing 34-de-(acetylamino-glucopyranosyl)-34-deoxy teicoplanin derivatives of the following formula I

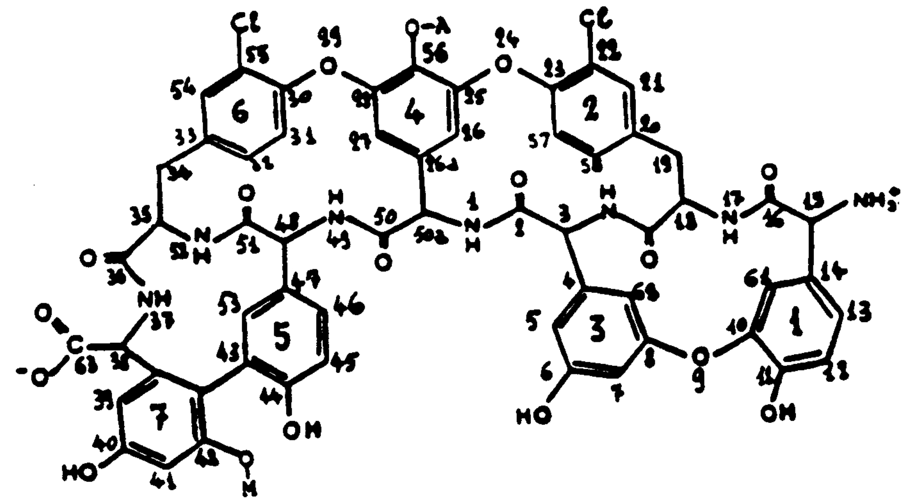

I

wherein

A represents hydrogen or OA represents N[$C_{10}$-$C_{11}$)aliphatic acyl]-beta-D-2-deoxy-2-amino-glucopyranosyl,

M represents hydrogen or OM represents alpha-D-mannopyranosyl, with the proviso that when M represents hydrogen also A must represent hydrogen,

and the addition salts thereof, which includes selectively removing the n-acetyl-beta-D-2-deoxy-2-amino-glucopyranosyl group from a compound of formula II:

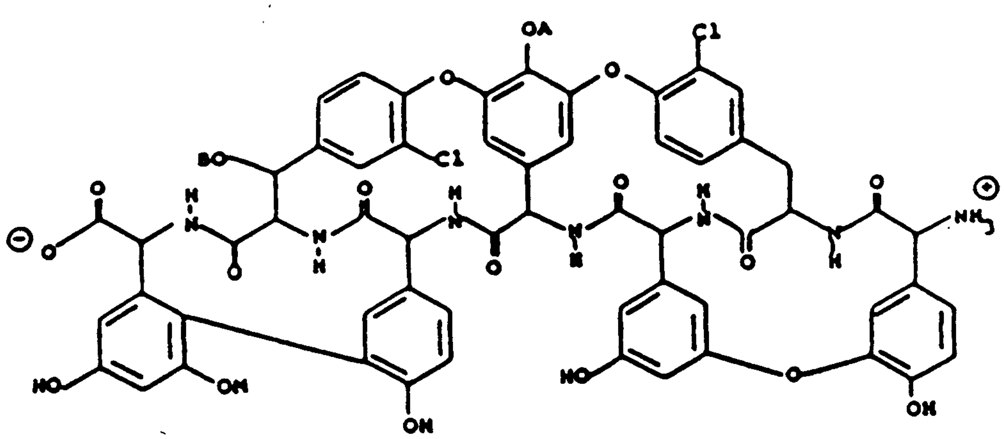

II

wherein A represents hydrogen or OA represents -N[$C_{10}$-$C_{11}$)aliphatic acyl]-beta-D-2-deoxy-2-aminoglycopyranosyl, B represents hydrogen or OB represents N-acetyl-beta-D-2-deoxy-2-aminoglucopyranosyl, M represents hydrogen or OM represents alpha-D-mannopyranosyl, with the proviso that B may represent hydrogen only when A and M are simultaneously hydrogen and M may represent hydrogen only when A is hydrogen, a salt thereof, or a mixture thereof, in any proportion, under conditions which do not affect the unsaturation on the ($C_{10}$-$C_{11}$)aliphatic acyl function.

**2.** A process according to claim 1 wherein the selective removal is conducted in the presence of a molar excess of an alkali metal or alkali-metal borohydride in a suitable medium.

**3.** A process according to claim 1 wherein the selective removal is conducted in the presence of an alkali metal or alkali-earth metal borohydride such as lithium, potassium, sodium or calcium borohydride or sodium cyanoborohydride.

**4.** A process according to claim 1 wherein the reaction medium is a mixture of a polar aprotic solvent and a polar protic solvent.

**5.** A process according to claim 4 wherein the reaction medium is a mixture of dimethylformamide and methanol, 1:1 (v/v).

**6.** A process according to claim 1 wherein the final product is treated with a mild reducing agent before separating it by known per se means from a more lipophilic by-product.

**7.** A process according to claim 6 wherein the mild reducing agent is represented by a molar excess of a metal capable of developing hydrogen from an aqueous acid solution or other redox agents having a similar standard reduction potential in the electrochemical series.

**8.** A process according to claim 6 wherein the mild reducing agent is zinc or tin in an aqueous mineral acid.

**9.** A process according to claim 6 wherein the reaction temperature is room temperature.

**10.** A process according to claim 6 wherein the separation of the final product is made by column chromatography.

**11.** A process for preparing a compound of claim 1, with the exclusion of a compound wherein OA represents beta-D-2-deoxy-2-((Z)-4-decenoyl)aminoglucopyranosyl, which comprises catalytically hydrogenating a compound of formula III:

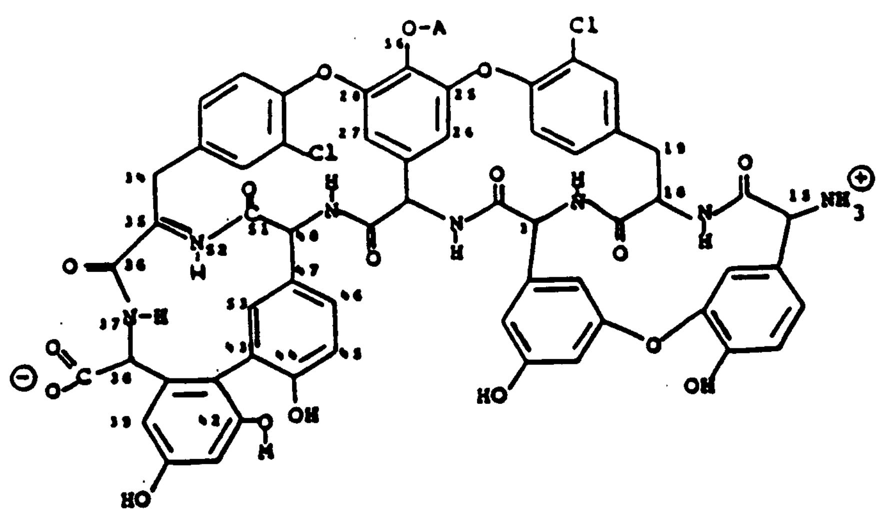

III

wherein,

A represents hydrogen or OA represents N[($C_{10}$-$C_{11}$)aliphatic acyl]-beta-D-2-deoxy-2-aminoglucopyranosyl,

M represents hydrogen or OM represents alpha-D-mannopyranosyl, with the proviso that when M represents hydrogen also A must represent hydrogen

and the addition salts thereof, in an aqueous alcoholic medium.

**12.** A process according to claim 11 wherein the reaction temperature is room temperature and the pressure is ambient pressure.

**13.** A process according to claim 11 wherein the hydrogenation catalyst is a transition metal or a transition metal derivative optionally on a suitable support.

**14.** A process according to claim 11 wherein the hydrogenation catalyst is selected from palladium on barium sulphate, palladium on charcoal, palladium on alumina, palladium on calcium carbonate, platinum, platinum oxide and rhodium on charcoal.

**15.** A process according to claim 11 wherein the hydrogenation catalyst is selected from 5% palladium on charcoal, 10% palladium on charcoal, 10% palladium on barium sulphate, 5% palladium on calcium carbonate and 5% rhodium on charcoal.

**16.** A process according to any of the preceding claims for preparing a compound wherein OA and OM represent the sugar moieties defined therein.

**17.** A process according to any of the preceding claims for preparing a compound wherein OA represents beta-D-2-deoxy-2-(8-methylnonanoyl)aminoglucopyranosyl and OM represents alpha-D-mannopyranosyl.

**18.** A process according to any of the preceding claims for preparing a compound wherein A and M represent hydrogen.

**19.** Use of a compound of claims 1, 16, 17 or 18 for preparing a medicament for antimicrobial use.

**Patentansprüche**
**Patentansprüche für folgende Vertragsstaaten : AT, BE, CH, DE, FR, GB, IT, LI, LU, NL, SE**

**1.** 34-Des-(acetylaminoglucopyranosyl)-34-desoxyteicoplanin-Derivate der folgenden Formel I

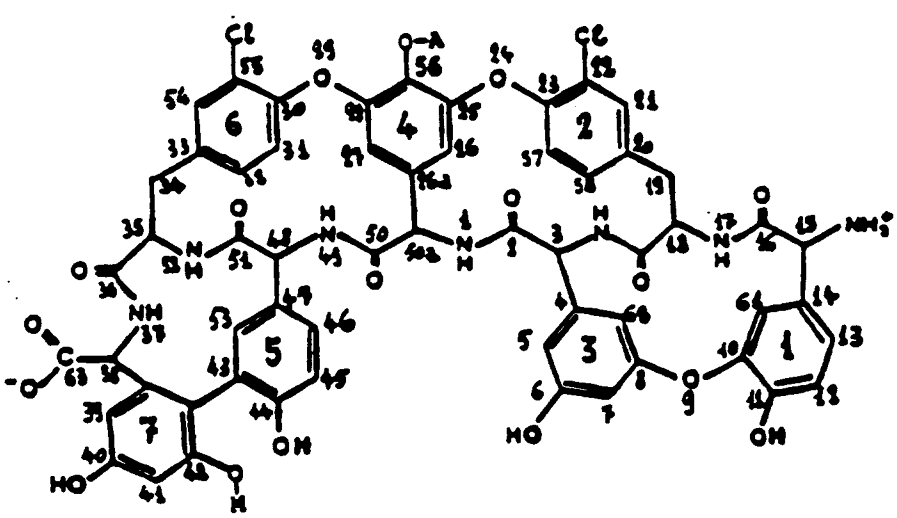

(I)

wobei

A ein Wasserstoffatom bedeutet oder OA einen N[($C_{10}$-$C_{11}$)-aliphatisch Acyl]-$\beta$-D-2-desoxy-2-amino-glucopyranosylrest bedeutet,

M ein Wasserstoffatom bedeutet oder OM eine α-D-Mannopyranosylgruppe bedeutet, mit der Maßgabe, daß, wenn M ein Wasserstoffatom bedeutet, A ebenfalls ein Wasserstoffatom sein muß, und die Additionssalze davon.

**2.** Verbindung nach Anspruch 1, wobei OA und OM die vorstehend definierten Zuckereinheiten bedeuten.

**3.** Verbindung nach Anspruch 1, wobei OA eine β-D-2-Desoxy2-(8-methylnonanoyl)-aminoglucopyranosylgruppe und OM eine α-D-Mannopyranosylgruppe bedeutet.

**4.** Verbindung nach Anspruch 1, wobei A und M ein Wasserstoffatom bedeuten.

**5.** Verfahren zur Herstellung einer Verbindung nach Anspruch 1 mit Ausnahme einer Verbindung, in der OA eine β-D-2-Desoxy-2-((Z)-4-decenoyl)aminoglucopyranosylgruppe bedeutet, umfassend die katalytische Hydrierung einer Verbindung der Formel III

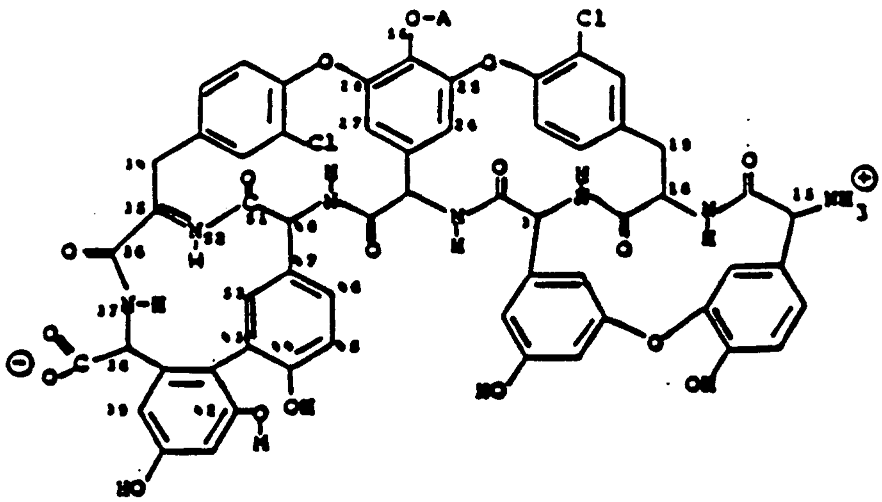

(III)

wobei
A ein Wasserstoffatom bedeutet, oder OA einen N[($C_{10}$-$C_{11}$)aliphatisch Acyl]-β-D-2-desoxy-2-amino-glucopyranosylrest bedeutet,
M ein Wasserstoffatom bedeutet, oder OM eine α-D-Mannopyranosylgruppebedeutet, mit der Maßgabe, daß, wenn M ein Wasserstoffatom bedeutet, A ebenfalls ein Wasserstoffatom sein muß,
und die Additionssalze davon,
in einem wäßrigen alkoholischen Medium.

**6.** Verfahren nach Anspruch 5, wobei die Umsetzungstemperatur Raumtemperatur und der Druck Umgebungsdruck ist.

**7.** Verfahren nach Anspruch 5, wobei der Hydrierkatalysator ein Übergangsmetall oder ein Übergangsmetallderivat gegebenenfalls auf einem geeigneten Träger ist.

**8.** Verfahren nach Anspruch 5, wobei der Hydrierkatalysator aus Palladium auf Bariumsulfat, Palladium auf Kohle, Palladium auf Aluminiumoxid, Palladium auf Calciumcarbonat, Platin, Platinoxid und Rhodium auf Kohle ausgewählt ist.

**9.** Verfahren zur Herstellung einer Verbindung nach Anspruch 1, umfassend das selektive Entfernen des N-Acetyl-β-D-2-Deoxy-aminoglucopyranosylrestes von einer Verbindung der Formel II

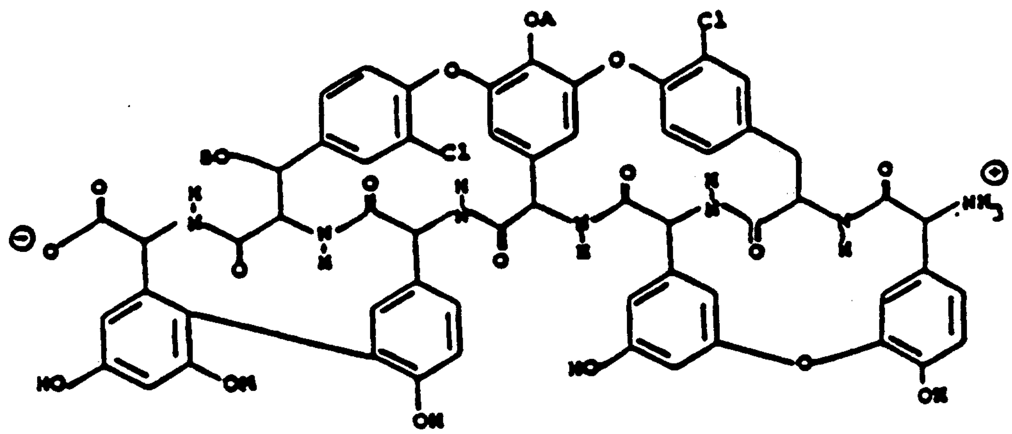 (II)

wobei A ein Wasserstoffatom bedeutet oder OA einen N[($C_{10}$-$C_{11}$)aliphatisch Acyl]-β-D-2-desoxy-aminoglucopyranosylrest bedeutet, B ein Wasserstoffatom bedeutet, oder OB einen N-Acetyl-β-D-2-desoxy-aminoglucopyranosylrest bedeutet, M ein Wasserstoffatom bedeutet, oder OM eine α-D-Mannopyranosylgruppe bedeutet, mit der Maßgabe, daß B nur dann ein Wasserstoffatom sein kann, wenn A und M beide Wasserstoffatome sind und M nur dann ein Wasserstoffatom sein kann, wenn A ein Wasserstoffatom ist, einem Salz davon oder einem Gemisch davon in beliebigen Anteilen unter Bedingungen beinhaltet, die die Ungesättigtheit der ($C_{10}$-$C_{11}$)aliphatisch Acylfunktion nicht beeinträchtigen.

**10.** Verfahren nach Anspruch 9, wobei die selektive Entfernung in Gegenwart eines molaren Überschusses eines Alkalimetalls oder eines Alkalimetallborhydrids in einem geeigneten Medium durchgeführt wird.

**11.** Verfahren nach Anspruch 9, wobei das Umsetzungsmedium ein Gemisch aus einem polar-aprotischen Lösungsmittel und einem polar-protischen Lösungsmittel ist.

**12.** Verfahren nach Anspruch 11, wobei das Umsetzungsmedium ein Gemisch von Dimethylformamid und Methanol, 1:1 (v/v) ist.

**13.** Verfahren nach Anspruch 9, wobei das Endprodukt mit einem schwachen Reduktionsmittel behandelt wird, bevor es in an sich bekannter Weise von einem stärker lipophilen Nebenprodukt abgetrennt wird.

**14.** Verfahren nach Anspruch 13, wobei das schwache Reduktionsmittel durch einen molaren Überschuß eines Metalls dargestellt wird, das befähigt ist, Wasserstoff von einer Lösung einer wäßrigen Säure oder anderen Redoxmitteln, die ein ähnliches Standard-Redoxpotential in der elektrochemischen Reihe haben, zu entwickeln.

**15.** Verfahren nach Anspruch 13, wobei das schwache Reduktionsmittel Zink oder Zinn in einer wäßrigen Mineralsäure ist.

**16.** Verfahren nach Anspruch 13, wobei die Umsetzungstemperatur Raumtemperatur ist.

**17.** Verbindung nach Anspruch 1, 2, 3 oder 4 zur Verwendung als Arzneimittel.

**18.** Verwendung einer Verbindung nach Anspruch 1, 2, 3 oder 4 zur Herstellung eines Arzneimittels zur antimikrobiellen Verwendung.

**19.** Arzneimittel, enthaltend eine Verbindung nach Anspruch 1, 2, 3 oder 4 im Gemisch mit einem pharmazeutisch verträglichen Träger.

**Patentansprüche für folgende Vertragsstaaten : ES, GR**

1. Verfahren zur Herstellung von 34-Des-(acetylaminoglucopyranosyl)-34-desoxyteicoplanin-Derivaten der folgenden Formel I

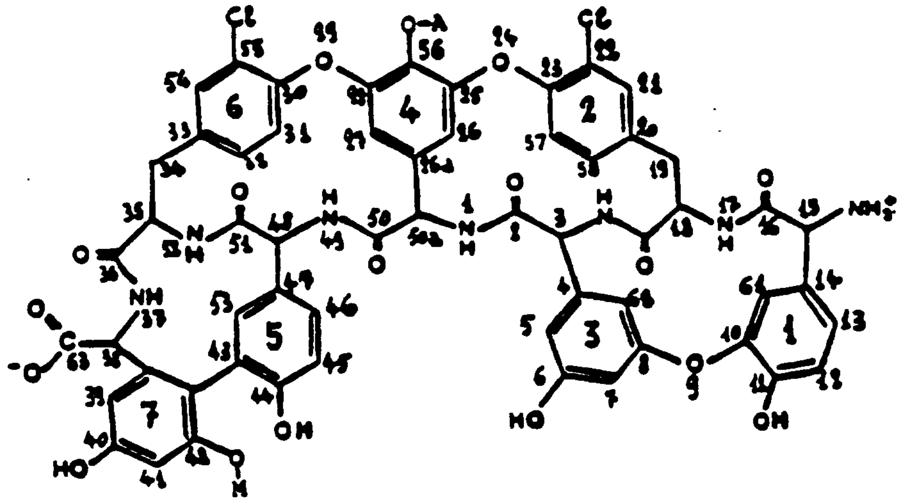

(I)

wobei
A ein Wasserstoffatom bedeutet oder OA einen N[($C_{10}$-$C_{11}$)-aliphatisch Acyl]-$\beta$-D-2-desoxy-2-amino-glucopyranosylrest bedeutet,
M ein Wasserstoffatom bedeutet oder OM eine $\alpha$-D-Mannopyranosylgruppe bedeutet, mit der Maßgabe, daß, wenn M ein Wasserstoffatom bedeutet, A ebenfalls ein Wasserstoffatom sein muß,
und die Additionssalze davon, umfassend das selektive Entfernen des N-Acetyl-$\beta$-D-2-Deoxy-aminoglucopyranosylrestes von einer Verbindung der Formel II

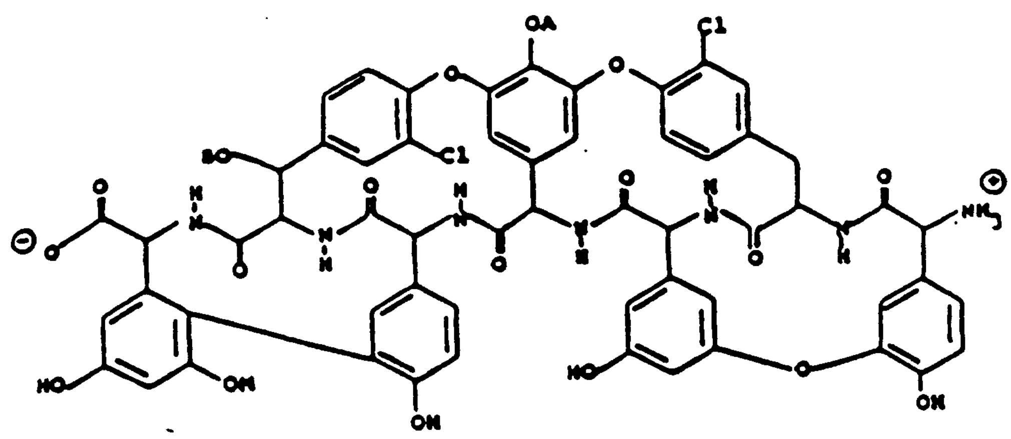

(II)

wobei A ein Wasserstoffatom bedeutet oder OA einen N[($C_{10}$-$C_{11}$)aliphatisch Acyl]-$\beta$-D-2-desoxy-aminoglucopyranosylrest bedeutet, B ein Wasserstoffatom bedeutet, oder OB einen N-Acetyl-$\beta$-D-2-desoxy-aminoglucopyranosylrest bedeutet, M ein Wasserstoffatom bedeutet, oder OM eine $\alpha$-D-Mannopyranosylgruppe bedeutet, mit der Maßgabe, daß B nur dann ein Wasserstoffatom sein kann, wenn A und M beide Wasserstoffatome sind und M nur dann ein Wasserstoffatom sein kann, wenn A ein Wasserstoffatom ist, einem Salz davon oder einem Gemisch davon in beliebigen Anteilen unter Bedingungen beinhaltet, die die Ungesättigtheit der ($C_{10}$-$C_{11}$)aliphatisch Acylfunktion nicht beeinträchtigen.

2. Verfahren nach Anspruch 1, wobei die selektive Entfernung in Gegenwart eines molaren Überschusses eines Alkalimetalls oder eines Alkalimetallborhydrids in einem geeigneten Medium durchgeführt wird.

3. Verfahren nach Anspruch 1, wobei die selektive Entfernung in Gegenwart eines Alkalimetall- oder eines Erdalkalimetallborhydrids, wie Lithium-, Kalium-, Natrium- oder Calciumborhydrid oder Natriumcyanborhydrid durchgeführt wird.

4. Verfahren nach Anspruch 1, wobei das Umsetzungsmedium ein Gemisch aus einem polar-aprotischen Lösungsmittel und einem polar-protischen Lösungsmittel ist.

5. Verfahren nach Anspruch 4, wobei das Umsetzungsmedium ein Gemisch von Dimethylformamid und Methanol, 1:1 (v/v) ist.

6. Verfahren nach Anspruch 1, wobei das Endprodukt mit einem schwachen Reduktionsmittel behandelt wird, bevor es in an sich bekannter Weise von einem stärker lipophilen Nebenprodukt abgetrennt wird.

7. Verfahren nach Anspruch 6, wobei das schwache Reduktionsmittel durch einen molaren Überschuß eines Metalls dargestellt wird, das befähigt ist, Wasserstoff von einer Lösung einer wäßrigen Säure oder anderen Redoxmitteln, die ein ähnliches Standard-Redoxpotential in der elektrochemischen Reihe haben, zu entwickeln.

8. Verfahren nach Anspruch 6, wobei das schwache Reduktionsmittel Zink oder Zinn in einer wäßrigen Mineralsäure ist.

9. Verfahren nach Anspruch 6, wobei die Umsetzungstemperatur Raumtemperatur ist.

10. Verfahren nach Anspruch 6, wobei die Abtrennung des Endproduktes mittels Säulenchromatographie erfolgt.

11. Verfahren zur Herstellung einer Verbindung von Anspruch 1 mit Ausnahme einer Verbindung, in der OA eine $\beta$-D-2-Desoxy-2-((Z)-4-decenoyl)aminoglucopyranosylgruppe bedeutet, umfassend die katalytische Hydrierung einer Verbindung der Formel III

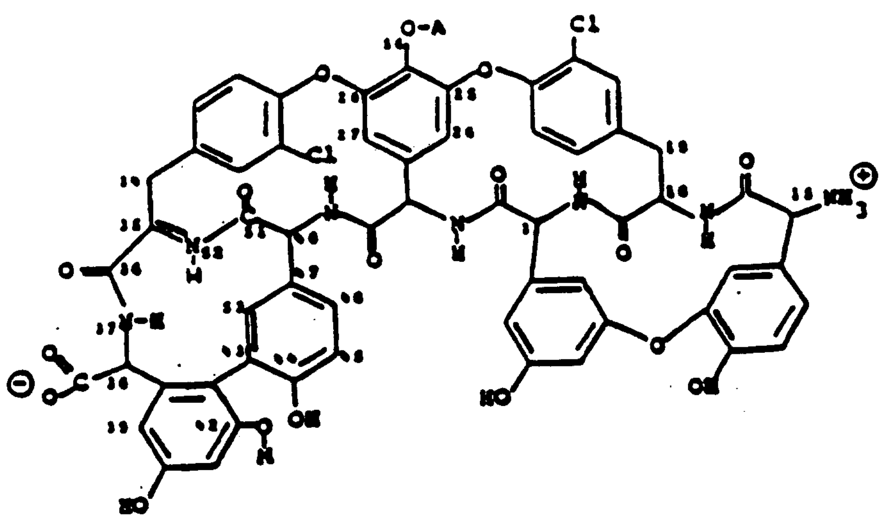

(III)

wobei
A ein Wasserstoffatom bedeutet, oder OA einen N[($C_{10}$-$C_{11}$)aliphatisch Acyl]-$\beta$-D-2-desoxy-2-aminoglucopyranosylrest bedeutet,
M ein Wasserstoffatom bedeutet, oder OM eine $\alpha$-D-Mannopyranosylgruppebedeutet, mit der Maßgabe, daß, wenn M ein Wasserstoffatom bedeutet, A ebenfalls ein Wasserstoffatom sein muß,
und die Additionssalze davon,
in einem wäßrigen alkoholischen Medium.

**12.** Verfahren nach Anspruch 11, wobei die Umsetzungstemperatur Raumtemperatur und der Druck Umgebungsdruck ist.

**13.** Verfahren nach Anspruch 11 wobei der Hydrierkatalysator ein Übergangsmetall oder ein Übergangsmetallderivat gegebenenfalls auf einem geeigneten Träger ist.

**14.** Verfahren nach Anspruch 11, wobei der Hydrierkatalysator aus Palladium auf Bariumsulfat, Palladium auf Kohle, Palladium auf Aluminiumoxid, Palladium auf Calciumcarbonat, Platin, Platinoxid und Rhodium auf Kohle ausgewählt ist.

**15.** Verfahren nach Anspruch 11, wobei der Hydrierkatalysator aus 5 % Palladium auf Kohle, 10 % Palladium auf Kohle, 10 % Palladium auf Bariumsulfat, 5 % Palladium auf Calciumcarbonat und 5 % Rhodium auf Kohle ausgewählt ist.

**16.** Verfahren nach einem der vorstehenden Ansprüche zur Herstellung einer Verbindung, wobei OA und OM die vorstehend definierten Zuckereinheiten bedeuten.

**17.** Verfahren nach einem der vorstehenden Ansprüche zur Herstellung einer Verbindung, wobei OA eine $\beta$-D-2-Desoxy-2-(8-methylnonanoyl)aminoglucopyranosylgruppe und OM eine $\alpha$-D-Mannopyranosylgruppe bedeutet.

**18.** Verfahren nach einem der vorstehenden Ansprüche zur Herstellung einer Verbindung, wobei A und M ein Wasserstoffatom bedeuten.

**19.** Verwendung einer Verbindung der Ansprüche 1, 16, 17 oder 18 zur Herstellung eines Arzneimittels zur antimikrobiellen Verwendung.

**Revendications**

**Revendications pour les Etats contractants suivants : AT, BE, CH, DE, FR, GB, IT, LU, NL, SE, LI**

**1.** Dérivés de la 34-dés-(acétylaminoglucopyranosyl)-34-désoxy-téicoplanine répondant à la formule I

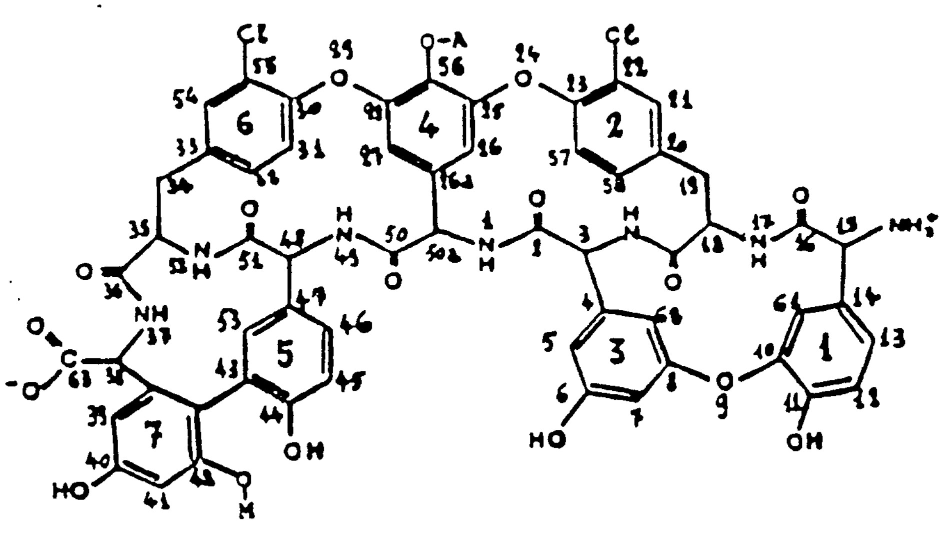

I

dans laquelle

A représente un atome d'hydrogène, ou OA représente un groupe N(acyle aliphatique en $C_{10}$-$C_{11}$)-bêta-D-2-désoxy-2-aminoglucopyranosyle,

M représente un atome d'hydrogène, ou OM représente un groupe alpha-D-mannopyranosyle, sous réserve que lorsque M représente un atome d'hydrogène, A doit lui aussi représenter un atome d'hydrogène,

et leurs sels d'addition.

2. Composé selon la revendication 1, dans lequel OA et OM représentent les parties sucre comme défini ici.

3. Composé selon la revendication 1, dans lequel OA représente un groupe bêta-D-2-désoxy-2-(8-méthylnonanoyl)-aminoglucopyranosyle et OM représente un groupe alpha-D-mannopyranosyle.

4. Composé selon la revendication 1, dans lequel A et M représentent des atomes d'hydrogène.

5. Procédé de préparation d'un composé selon la revendication 1, à l'exclusion d'un composé dans lequel OA représente un groupe bêta-D-2-désoxy-2-((Z)-4-décénoyl)-aminoglucopyranosyle, qui comprend le fait d'hydrogéner catalytiquement un composé répondant à la formule III :

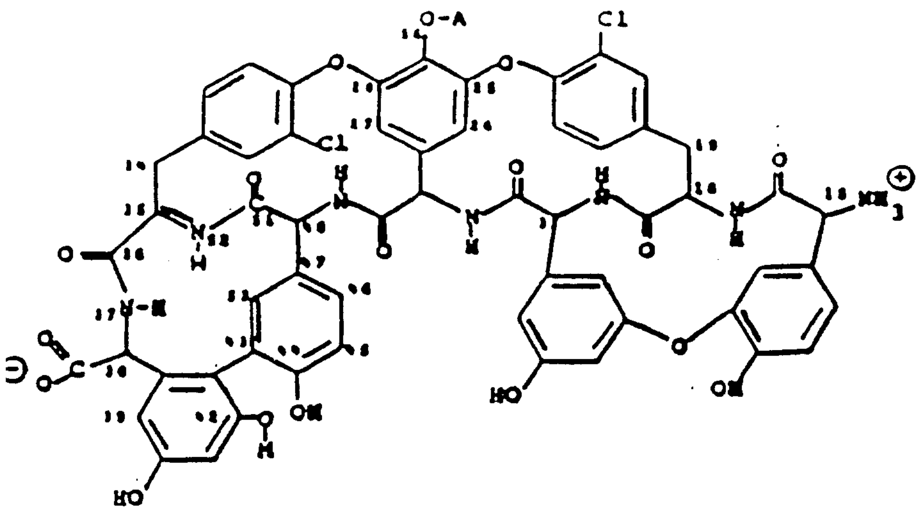

III

dans laquelle

A représente un atome d'hydrogène ou OA représente un groupe -N(acyle aliphatique en $C_{10}$-$C_{11}$)-bêta-D-2-désoxy-2-aminoglucopyranosyle,

M représente un atome d'hydrogène ou OM représente un groupe alpha-D-mannopyranosyle, sous réserve que, lorsque M représente un atome d'hydrogène, A doit aussi représenter un atome d'hydrogène,

et leurs sels d'addition dans un milieu alcoolique aqueux.

6. Procédé selon la revendication 5, dans lequel la température de réaction est la température ambiante et la pression est la pression ambiante.

7. Procédé selon la revendication 5, dans lequel le catalyseur d'hydrogénation est un métal de transition ou un dérivé de métal de transition, éventuellement sur un support approprié.

8. Procédé selon la revendication 5, dans lequel le catalyseur d'hydrogénation est choisi parmi le palladium sur sulfate de baryum, le palladium sur charbon, le palladium sur alumine, le palladium sur carbonate de calcium, le platine, l'oxyde de platine et le rhodium sur charbon.

9. Procédé pour préparer un composé selon la revendication 1, qui comprend le fait d'éliminer sélectivement le groupe N-acétyl-bêta-D-2-désoxy-2-amino-glucopyranosyle d'un composé répondant à la formule II ;

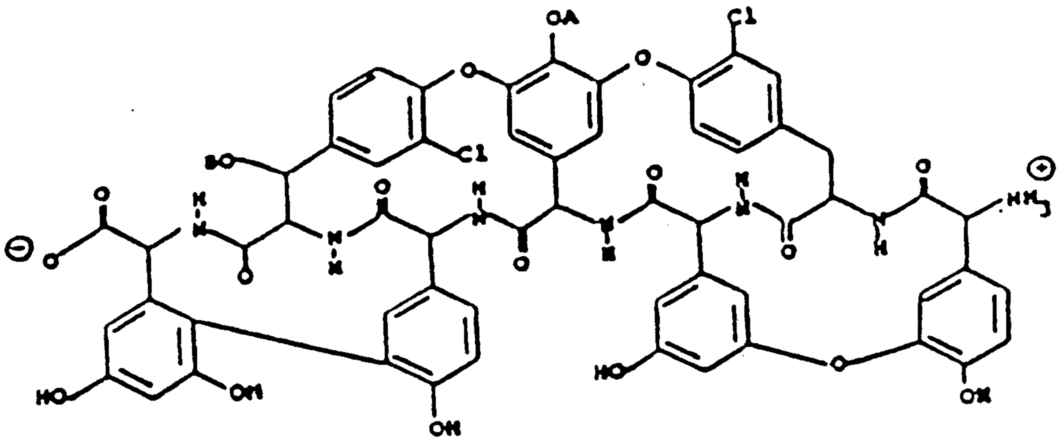

II

dans laquelle A représente un atome d'hydrogène ou OA représente un groupe -N(acyle aliphatique en $C_{10}$-$C_{11}$)-bêta-D-2-désoxy-2-aminoglucopyranosyle, B représente un atome d'hydrogène, ou OB représente un groupe N-acétyl-bêta-D-2-désoxy-2-aminoglucopyranosyle, M représente un atome d'hydrogène ou OM représente un groupe alpha-D-mannopyranosyle, sous réserve que B ne peut représenter un atome d'hydrogène que lorsque A et M sont simultanément des atomes d'hydrogène et que M ne peut représenter un atome d'hydrogène que lorsque A est un atome d'hydrogène, un de ses sels ou un mélange de ceux-ci, en proportion quelconque, dans des conditions qui n'affectent pas l'insaturation sur la fonction acyle aliphatique en $C_{10}$-$C_{11}$.

**10.** Procédé selon la revendication 9, dans lequel l'élimination sélective est effectuée en présence d'un excès molaire d'un borohydrure de métal alcalin ou alcalino-terreux dans un milieu approprié.

**11.** Procédé selon la revendication 9, dans lequel le milieu réactionnel est un mélange d'un solvant aprotique polaire et d'un solvant protique polaire.

**12.** Procédé selon la revendication 11, dans lequel le milieu réactionnel est un mélange de diméthylformamide et de méthanol, 1:1 (v/v).

**13.** Procédé selon la revendication 9, dans lequel le produit final est traité par un agent réducteur doux avant d'être séparé par des moyens connus en soi d'un sous-produit plus lipophile.

**14.** Procédé selon la revendication 13, dans lequel l'agent réducteur doux est représenté par un excès molaire d'un métal capable de faire dégager de l'hydrogène d'une solution acide aqueuse ou d'autres agents redox ayant un potentiel de réduction normal similaire dans la série électro-chimique.

**15.** Procédé selon la revendication 13, dans lequel l'agent réducteur doux est le zinc ou l'étain dans un acide minéral aqueux.

**16.** Procédé selon la revendication 13, dans lequel la température de réaction est la température ambiante.

**17.** Composé selon la revendication 1, 2, 3 ou 4, pour l'utilisation comme médicament.

**18.** Utilisation d'un composé selon la revendication 1, 2, 3 ou 4 pour la préparation d'un médicament à utilisation antimicrobienne.

**19.** Composition pharmaceutique qui contient un composé selon la revendication 1, 2, 3 ou 4 en mélange avec un support pharmaceutiquement acceptable.

**Revendications pour les Etats contractants suivants : ES, GR**

**1.** Procédé pour la préparation de dérivés de la 34-dés-(acétylaminoglucopyranosyl)-34-désoxy-téicoplanine répondant à la formule I

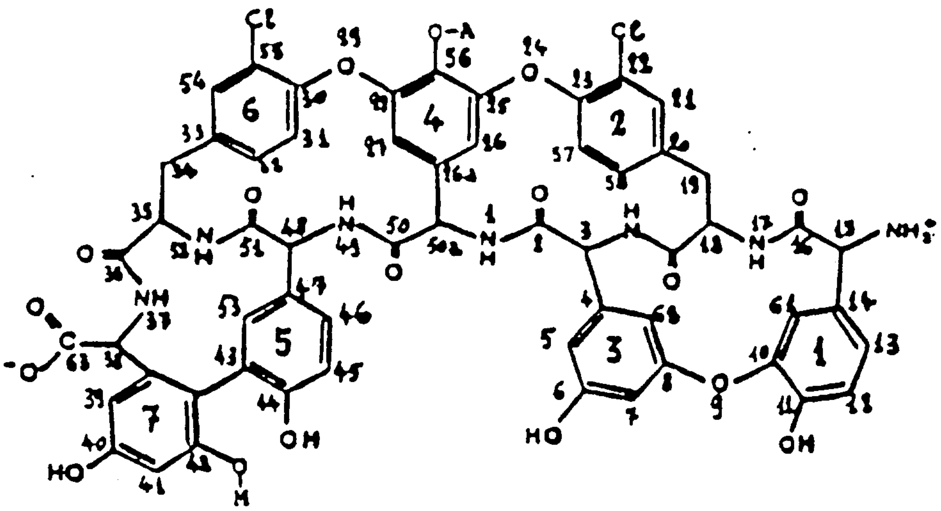

I

dans laquelle

A représente un atome d'hydrogène, ou OA représente un groupe N(acyle aliphatique en $C_{10}$-$C_{11}$)-bêta-D-2-désoxy-2-aminoglucopyranosyle,

M représente un atome d'hydrogène, ou OM représente un groupe alpha-D-mannopyranosyle, sous réserve que, lorsque M représente un atome d'hydrogène, A doit lui aussi représenter un atome d'hydrogène,

et leurs sels d'addition,

qui comprend le fait d'éliminer sélectivement le groupe N-acétyl-bêta-D-2-désoxy-2-aminoglucopyranosyle d'un composé répondant à la formule II :

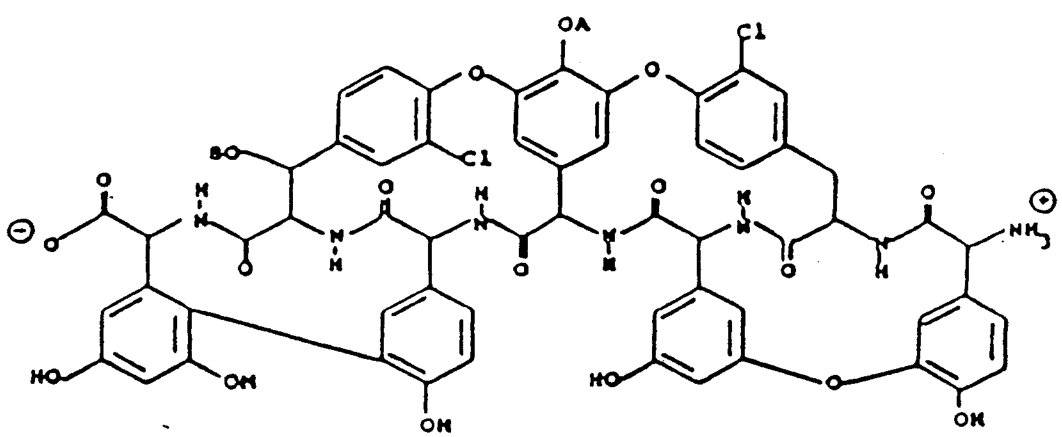

II

dans laquelle A représente un atome d'hydrogène ou OA représente un groupe -N(acyle aliphatique en $C_{10}$-$C_{11}$)-bêta-D-2-désoxy-2-aminoglucopyranosyle, B représente un atome d'hydrogène, ou OB représente un groupe N-acétyl-bêta-D-2-désoxy-2-aminoglucopyranosyle, M représente un atome d'hydrogène ou OM représente un groupe alpha-D-mannopyranosyle, sous réserve que B ne peut représenter un atome d'hydrogène que lorsque A et M sont simultanément des atomes d'hydrogène et que M ne peut représenter un atome d'hydrogène que lorsque A est un atome d'hydrogène, un de leurs sels ou un mélange de ceux-ci, en proportion quelconque, dans des conditions qui n'affectent pas l'insaturation sur la fonction acyle aliphatique en $C_{10}$-$C_{11}$.

**2.** Procédé selon la revendication 1, dans lequel l'élimination sélective est effectuée en présence d'un d'un excès molaire d'un borohydrure de métal alcalin ou de métal alcalino-terreux dans un milieu convenable.

**3.** Procédé selon la revendication 1, dans lequel l'élimination sélective est effectuée en présence d'un borohydrure de métal alcalin ou de métal alcalino-terreux tel que les borohydrures de lithium, de potassium, de sodium ou de calcium, ou le cyanoborohydrure de sodium.

**4.** Procédé selon la revendication 1, dans lequel le milieu réactionnel est un mélange d'un solvant aprotique polaire et d'un solvant protique polaire.

**5.** Procédé selon la revendication 4, dans lequel le milieu réactionnel est un mélange de diméthylformamide et de méthanol, 1:1 (v/v).

**6.** Procédé selon la revendication 1, dans lequel le produit final est traité par un agent réducteur doux avant d'être séparé, par des moyens connus en soi, d'un sous-produit plus lipophile.

**7.** Procédé selon la revendication 6, dans lequel l'agent réducteur doux est représenté par un excès molaire d'un métal capable de faire dégager de l'hydrogène d'une solution acide aqueuse ou d'autres agents redox ayant un potentiel de réduction normal similaire dans la série électro-chimique.

**8.** Procédé selon la revendication 6, dans lequel l'agent réducteur doux est le zinc ou l'étain dans un acide minéral aqueux.

**9.** Procédé selon la revendication 6, dans lequel la température de réaction est la température ambiante.

**10.** Procédé selon la revendication 6, dans lequel la séparation du produit final est effectuée par chromatographie sur colonne.

**11.** Procédé de préparation d'un composé selon la revendication 1, à l'exclusion d'un composé dans lequel OA représente un groupe bêta-D-2-désoxy-2-((Z)-4-décénoyl)-aminoglucopyranosyle, qui comprend le fait d'hydrogéner catalytiquement un composé répondant à la formule III :

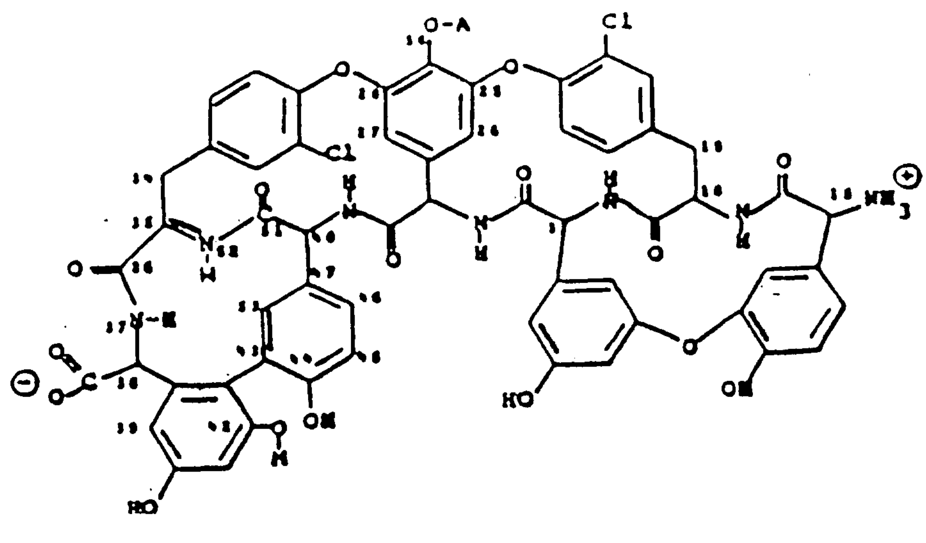

III

dans laquelle

A représente un atome d'hydrogène ou OA représente un groupe -N(acyle aliphatique en $C_{10}$-$C_{11}$)-bêta-D-2-désoxy-2-aminoglucopyranosyle,

M représente un atome d'hydrogène ou OM représente un groupe alpha-D-mannopyranosyle, sous réserve que, lorsque M représente un atome d'hydrogène, A doit aussi représenter un atome d'hydrogène,

et ses sels d'addition dans un milieu alcoolique aqueux.

**12.** Procédé selon la revendication 11, dans lequel la température de réaction est la température ambiante et la pression est la pression ambiante.

**13.** Procédé selon la revendication 11, dans lequel le catalyseur d'hydrogénation est un métal de transition ou un dérivé de métal de transition, éventuellement sur un support approprié.

**14.** Procédé selon la revendication 11, dans lequel le catalyseur d'hydrogénation est choisi parmi le palladium sur sulfate de baryum, le palladium sur charbon, le palladium sur alumine, le palladium sur carbonate de calcium, le platine, l'oxyde de platine et le rhodium sur charbon.

**15.** Procédé selon la revendication 11 dans lequel le catalyseur d'hydrogénation est choisi parmi le palladium à 5 % sur charbon, le palladium à 10 % sur charbon, le palladium à 10 % sur sulfate de baryum, le palladium à 5 % sur carbonate de calcium et le rhodium à 5 % sur charbon.

**16.** Procédé selon l'une quelconque des revendications précédentes pour préparer un composé dans lequel OA et OM représentent les parties sucre qui y sont définies.

**17.** Procédé selon l'une quelconque des revendications précédentes pour préparer un composé dans lequel OA représente un groupe bêta-D-2-désoxy-2-(8-méthylnonanoyl)aminoglucopyranosyle et OM représente un groupe alpha-D-mannopyranosyle.

**18.** Procédé selon l'une quelconque des revendications précédentes pour préparer un composé dans lequel A et M représentent des atomes d'hydrogène.

**19.** Utilisation d'un composé selon la revendication 1, 16, 17 ou 18 pour préparer un médicament à utilisation antimicrobienne.